# EUROPEAN PATENT SPECIFICATION

(11) **EP 3 861 347 B1**
(45) Date of publication and mention of the grant of the patent: **21.12.2022**
(21) Application number: 19790312.3
(22) Date of filing: 02.10.2019
(51) Int. Cl.: G01N 33/574, G01N 33/68, G01N 33/74

(54) **BIOMARKERS FOR A COMBINATION THERAPY COMPRISING LENVATINIB AND EVEROLIMUS**
BIOMARKER FÜR EINE KOMBINATIONSTHERAPIE MIT LENVATINIB UND EVEROLIMUS
BIOMARQUEURS POUR UNE POLYTHÉRAPIE COMPRENANT DU LENVATINIB ET DE L'ÉVÉROLIMUS

(30) Priority: 05.10.2018 US 201862741861 P
(43) Date of publication of application: 11.08.2021
(73) Proprietor: Eisai R&D Management Co., Ltd., Tokyo 112-8088 (JP)
(72) Inventor: FUNAHASHI Yasuhiro, Tsukuba-shi, Ibaraki 300-2635 (JP); MINOSHIMA Yukinori, Tsukuba-shi, Ibaraki 300-2635 (JP); KANEKIYO Michio, Woodcliff Lake,New Jersey 07677 (US); MATSUKI Masahiro, Tsukuba-shi, Ibaraki 300-2635 (JP); ADACHI Yusuke, Tsukuba-shi, Ibaraki 300-2635 (JP); KODAMA Kotaro, Tsukuba-shi, Ibaraki 300-2635 (JP)
(74) Representative: Hoffmann Eitle
(86) International application number: PCT/JP2019/039012
(87) International publication number: WO 2020/071457

(56) References cited:
- WO-A1-2016/194348
- H. GLEN ET AL: "432 Correlative analyses of serum biomarkers and clinical outcomes in the phase 2 study of lenvatinib, everolimus, and the combination, in patients with metastatic renal cell carcinoma following 1 VEGF-targeted therapy", EUROPEAN JOURNAL OF CANCER, vol. 51, 1 September 2015 (2015-09-01), page S89, XP055510094, AMSTERDAM, NL ISSN: 0959-8049, DOI: 10.1016/S0959-8049(16)30266-0
- C-H LEE ET AL: "76P Correlative analyses of serum biomarkers and efficacy outcomes in the randomized phase II trial of lenvatinib (LEN), everolimus (EVE), or LEN+EVE in patients with metastatic renal cell carcinoma", 43RD CONGRESS OF EUROPEAN SOCIETY FOR MEDICAL ONCOLOGY, ESMO 2018, vol. 29(Sup8), 23 October 2018 (2018-10-23), page viii23, XP055663674, DOI: 10.1093/annonc/mdy269

## Description

### [Technical Field]

The present invention relates to predicting the response of a human subject having, suspected of having, or at risk of developing a renal cell carcinoma to a combination therapy comprising lenvatinib and everolimus.

### [Background Art]

Renal cell carcinoma (RCC) is the most common type of kidney cancer in adults, which consists of about 90 percent of diagnosed kidney cancers.

Lenvatinib mesylate has been approved as LENVIMA^{(Registered Trademark)} by the U.S. Food and Drug Administration for the treatment of patients with locally recurrent or metastatic, progressive, radioactive iodine-refractory differentiated thyroid cancer, unresectable hepatocellular carcinoma, or in combination with everolimus, for the treatment of patients with advanced renal cell carcinoma.

Most anti-tumor treatments are associated with undesirable side effects, such as profound nausea, vomiting, or severe fatigue. Such side effects need to be controlled especially when multiple anti-tumor agents are used as a combination therapy. Also, while anti-tumor treatments have been successful, they do not produce significant clinical responses in all patients who receive them, resulting in undesirable side effects, delays, and costs associated with ineffective treatment. Therefore, biomarkers that can be used to predict the response of a subject to an antitumor agent, especially when it is used in a combination therapy, prior to administration thereof are greatly needed. In WO 2016/194348, for example, a method of predicting the response of a human subject having, suspected of having, or at risk of developing a renal cell carcinoma to a combination therapy comprising lenvatinib or a pharmaceutically acceptable salt thereof and everolimus is described, which comprises measuring the expression level of angiopoietin-2 (ANG2) in a patient's biological sample. In Glen et al. Eur J Cancer, Vol. 51, Supplement 3, S89, 1 September 2015, the measurement of serum levels of VEGF, FGF-23 and ANG-2 to identify responders to the combination therapy with lenvatinib and everolimus is described.

### [Summary of Invention]

The present invention is set out in the appended set of claims. It is based, at least in part, on the identification of biomarkers that can be used to identify or select a human subject having, suspected of having, or at risk of developing, a renal cell carcinoma responsive to a combination therapy comprising lenvatinib or a pharmaceutically acceptable salt thereof (e.g., lenvatinib mesylate) and everolimus. The expression level of at least one protein selected from the group consisting of IL-18BP, ICAM-1, FGF-21 and M-CSF in a biological sample obtained from the human subject prior to treatment ("baseline level") is identified as a useful predictor of a human subject having, suspected of having, or at risk of developing, a renal cell carcinoma responsive to a combination therapy comprising lenvatinib or a pharmaceutically acceptable salt thereof (e.g., lenvatinib mesylate) and everolimus. In addition, a composite biomarker based on the expression level of at least five proteins comprising (i) HGF, MIG, IL-18BP, IL-18 and ANG-2, or (ii) TIMP-1, M-CSF, IL-18BP, ANG-2 and VEGF-A in a biological sample obtained from the human subject prior to treatment ("baseline level") is identified as a useful predictor of a human subject having, suspected of having, or at risk of developing, a renal cell carcinoma responsive to a combination therapy comprising lenvatinib or a pharmaceutically acceptable salt thereof (e.g., lenvatinib mesylate) and everolimus. Furthermore, a composite biomarker based on the expression level of at least two proteins comprising IL-18BP and ANG-2 in a biological sample obtained from the human subject prior to treatment ("baseline level") is identified as a useful predictor of a human subject having, suspected of having, or at risk of developing, a renal cell carcinoma responsive to a combination therapy comprising lenvatinib or a pharmaceutically acceptable salt thereof (e.g., lenvatinib mesylate) and everolimus. Thus, the biomarkers including the composite biomarker score and compositions described herein are useful, for example, in identifying, stratifying, and/or selecting a patient or a subset of patients having renal cell carcinoma that could benefit from a combination therapy comprising lenvatinib or a pharmaceutically acceptable salt thereof (e.g., lenvatinib mesylate) and everolimus. In addition, the methods described herein are useful, for example, in selecting appropriate treatment modalities (e.g., combination therapy comprising lenvatinib or a pharmaceutically acceptable salt thereof (e.g., lenvatinib mesylate) and everolimus or an alternative renal cell carcinoma therapy) for a subject suffering from, suspected of having, or at risk of developing a renal cell carcinoma. Where in the present description reference is made to methods of treatment, this is to be interpreted as reference to the compounds, pharmaceutical compositions or medicaments of the present invention for use in the respective methods of treatment.

In one aspect, the disclosure provides a method of identifying a human subject having, suspected of having, or at risk of developing, a renal cell carcinoma responsive to a combination therapy comprising lenvatinib or a pharmaceutically acceptable salt thereof and everolimus. The method involves assaying a biological sample obtained from the subject before administration of the combination therapy and determining that the expression level of at least one protein selected from the group consisting of IL-18BP, ICAM-1, FGF-21 and M-CSF in the biological sample is lower, as compared to a control. The subject having a low expression level of at least one protein selected from the group consisting of IL-18BP, ICAM-1, FGF-21 and M-CSF in the biological sample is identified as responsive to the combination therapy comprising lenvatinib or a pharmaceutically acceptable salt thereof and everolimus. Alternatively, the method involves assaying a biological sample obtained from the subject before administration of the combination therapy and determining that the composite biomarker score calculated based on the baseline expression levels of
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2 in the biological sample is within a range that indicates that the human subject is responsive to the combination therapy comprising lenvatinib or a pharmaceutically acceptable salt thereof and everolimus, as compared to a control composite marker score. The subject having such a composite biomarker score in the biological sample is identified as responsive to the combination therapy. In certain embodiments, the composite biomarker score is calculated based on the baseline expression levels of at least five proteins comprising (i) HGF, MIG, IL-18BP, IL-18 and ANG-2, or (ii) TIMP-1, M-CSF, IL-18BP, ANG-2 and VEGF-A in the biological sample. In some embodiments, the composite biomarker score is calculated based on the baseline expression levels of at least five proteins comprising HGF, MIG, IL-18BP, IL-18 and ANG-2 in the biological sample. In some embodiments, the composite biomarker score is calculated based on the baseline expression levels of at least five proteins comprising TIMP-1, M-CSF, IL-18BP, ANG-2 and VEGF-A in the biological sample. In other embodiments, the composite biomarker score is calculated based on the baseline expression levels of at least two proteins comprising IL-18BP and ANG-2 in the biological sample.

In a second aspect, the disclosure features a method of selecting a human subject having, suspected of having, or at risk of developing, a renal cell carcinoma for administration with a combination therapy comprising lenvatinib or a pharmaceutically acceptable salt thereof and everolimus. The method comprises assaying a biological sample obtained from the human subject for the baseline expression level of at least one protein selected from the group consisting of IL-18BP, ICAM-1, FGF-21 and M-CSF. If it is determined that the baseline expression level of at least one protein selected from the group consisting of IL-18BP, ICAM-1, FGF-21 and M-CSF in the biological sample is lower, as compared to a control, the subject is selected for administration of the combination therapy. In certain embodiments, the method further comprises administering the combination therapy to the human subject. Alternatively, the method comprises assaying a biological sample obtained from the human subject for the baseline expression level of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   in the biological sample. If it is determined that the composite biomarker score is within a range that indicates that the human subject is responsive to the combination therapy, as compared to a control composite marker score, the subject is selected for administration of the combination therapy. In certain embodiments, the method further comprises administering the combination therapy to the human subject. In certain embodiments, the method comprises assaying the biological sample for the baseline expression level of at least five proteins comprising (i) HGF, MIG, IL-18BP, IL-18 and ANG-2, or (ii) TIMP-1, M-CSF, IL-18BP, ANG-2 and VEGF-A in the biological sample. In some embodiments, the method comprises assaying the biological sample for the baseline expression level of at least five proteins comprising HGF, MIG, IL-18BP, IL-18 and ANG-2 in the biological sample. In some embodiments, the method comprises assaying the biological sample for the baseline expression level of at least five proteins comprising TIMP-1, M-CSF, IL-18BP, ANG-2 and VEGF-A in the biological sample. In other embodiments, the method comprises assaying the biological sample for the baseline expression level of at least two proteins comprising IL-18BP and ANG-2 in the biological sample.

In a third aspect, the disclosure provides a method of treating a renal cell carcinoma. The method involves providing a biological sample obtained from a human subject having renal cell carcinoma before the treatment; measuring, in the biological sample, an expression level of at least one protein selected from the group consisting of IL-18BP, ICAM-1, FGF-21 and M-CSF that is lower as compared to a control; and administering to the human subject a therapeutically effective amount of lenvatinib or a pharmaceutically acceptable salt thereof and everolimus. Alternatively, the method comprises involves providing a biological sample obtained from a human subject having renal cell carcinoma before the treatment; measuring a composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   in the biological sample; and administering to the human subject a therapeutically effective amount of lenvatinib or a pharmaceutically acceptable salt thereof and everolimus, wherein the composite biomarker score indicates that the human subject is responsive to the combination therapy as compared to a control composite marker score. In certain embodiments, the composite biomarker score is calculated based on the baseline expression levels of at least five proteins comprising (i) HGF, MIG, IL-18BP, IL-18 and ANG-2, or (ii) TIMP-1, M-CSF, IL-18BP, ANG-2 and VEGF-A in the biological sample. In some embodiments, the composite biomarker score is calculated based on the baseline expression levels of at least five proteins comprising HGF, MIG, IL-18BP, IL-18 and ANG-2 in the biological sample. In some embodiments, the composite biomarker score is calculated based on the baseline expression levels of at least five proteins comprising TIMP-1, M-CSF, IL-18BP, ANG-2 and VEGF-A in the biological sample. In other embodiments, the composite biomarker score is calculated based on the baseline expression levels of at least two proteins comprising IL-18BP and ANG-2 in the biological sample.

In a fourth aspect, the disclosure provides a method of treating a renal cell carcinoma. The method involves administering to a human subject that has a renal cell carcinoma a therapeutically effective amount of a combination therapy comprising lenvatinib or a pharmaceutically acceptable salt thereof and everolimus, wherein the human subject has been identified as having an expression level of at least one protein selected from the group consisting of IL-18BP, ICAM-1, FGF-21 and M-CSF that is lower as compared to a control, or a composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   within a range that indicates that the human subject is responsive to the combination therapy comprising lenvatinib or a pharmaceutically acceptable salt thereof and everolimus, as compared to a control composite marker score. In certain embodiments, the subject has been identified as having a lower expression level of at least one protein selected from the group consisting of IL-18BP, ICAM-1, FGF-21 and M-CSF in a biological sample obtained from the human subject. In certain embodiments, the subject has been identified as having a composite biomarker score calculated based on the baseline expression levels of at least five proteins comprising (i) HGF, MIG, IL-18BP, IL-18 and ANG-2, or (ii) TIMP-1, M-CSF, IL-18BP, ANG-2 and VEGF-A in a biological sample obtained from the human subject, wherein the composite biomarker score within a range indicates that the human subject is responsive to the combination therapy as compared to a control composite marker score. In some embodiments, the subject has been identified as having a composite biomarker score calculated based on the baseline expression levels of at least five proteins comprising HGF, MIG, IL-18BP, IL-18 and ANG-2 in a biological sample obtained from the human subject, wherein the composite biomarker score within a range indicates that the human subject is responsive to the combination therapy as compared to a control composite marker score.

In some embodiments, the subject has been identified as having a composite biomarker score calculated based on the baseline expression levels of at least five proteins comprising TIMP-1, M-CSF, IL-18BP, ANG-2 and VEGF-A in a biological sample obtained from the human subject, wherein the composite biomarker score within a range indicates that the human subject is responsive to the combination therapy as compared to a control composite marker score. In some embodiments, the subject has been identified as having a composite biomarker score calculated based on the baseline expression levels of at least two proteins comprising IL-18BP and ANG-2 in a biological sample obtained from the human subject, wherein the composite biomarker score within a range indicates that the human subject is responsive to the combination therapy as compared to a control composite marker score.

In a fifth aspect, the disclosure features a method of treating a human subject having, suspected of having, or at risk of developing, a renal cell carcinoma. The method involves administering the human subject with a combination therapy comprising everolimus and lenvatinib or a pharmaceutically acceptable salt thereof, wherein the human subject has a baseline expression level of at least one protein selected from the group consisting of IL-18BP, ICAM-1, FGF-21 and M-CSF in a biological sample obtained from the subject that is lower than a control, or the human subject has a composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   within a range that indicates that the human subject is responsive to the combination therapy comprising lenvatinib or a pharmaceutically acceptable salt thereof and everolimus, as compared to a control composite marker score. In certain embodiments, the method involves administering the human subject with a combination therapy comprising everolimus and lenvatinib or a pharmaceutically acceptable salt thereof, wherein the human subject has a baseline expression level of at least one protein selected from the group consisting of IL-18BP, ICAM-1, FGF-21 and M-CSF in a biological sample obtained from the subject that is lower than a control. In some embodiments, the method involves administering the human subject with a combination therapy comprising everolimus and lenvatinib or a pharmaceutically acceptable salt thereof, wherein the human subject has a composite biomarker score calculated based on the baseline expression levels of at least five proteins comprising (i) HGF, MIG, IL-18BP, IL-18 and ANG-2, or (ii) TIMP-1, M-CSF, IL-18BP, ANG-2 and VEGF-A within a range that indicates that the human subject is responsive to the combination therapy comprising lenvatinib or a pharmaceutically acceptable salt thereof and everolimus, as compared to a control composite marker score. In some embodiments, the method involves administering the human subject with a combination therapy comprising everolimus and lenvatinib or a pharmaceutically acceptable salt thereof, wherein the human subject has a composite biomarker score calculated based on the baseline expression levels of at least five proteins comprising HGF, MIG, IL-18BP, IL-18 and ANG-2 within a range that indicates that the human subject is responsive to the combination therapy comprising lenvatinib or a pharmaceutically acceptable salt thereof and everolimus, as compared to a control composite marker score. In some embodiments, the method involves administering the human subject with a combination therapy comprising everolimus and lenvatinib or a pharmaceutically acceptable salt thereof, wherein the human subject has a composite biomarker score calculated based on the baseline expression levels of at least five proteins comprising TIMP-1, M-CSF, IL-18BP, ANG-2 and VEGF-A within a range that indicates that the human subject is responsive to the combination therapy comprising lenvatinib or a pharmaceutically acceptable salt thereof and everolimus, as compared to a control composite marker score. In other embodiments, the method involves administering the human subject with a combination therapy comprising everolimus and lenvatinib or a pharmaceutically acceptable salt thereof, wherein the human subject has a composite biomarker score calculated based on the baseline expression levels of at least two proteins comprising IL-18BP and ANG-2 within a range that indicates that the human subject is responsive to the combination therapy comprising lenvatinib or a pharmaceutically acceptable salt thereof and everolimus, as compared to a control composite marker score.

The composite biomarker score described above can be calculated by the steps of: measuring baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   in the biological sample;
   determining a score for each protein based on its baseline expression level; and
   summing up the scores to obtain a composite biomarker score.

In one embodiment, a larger value can be assigned as a score for each protein if a baseline expression level falls within a value range that is correlated with a population of human subjects with better therapeutic outcomes in response to the combination therapy, where a composite biomarker score equal to or higher than the control composite biomarker score is predictive that the human subject is responsive to the combination therapy comprising lenvatinib or a pharmaceutically acceptable salt thereof and everolimus. In one embodiment, a smaller value can be assigned as a score for each protein if a baseline expression level falls within a value range that is correlated with a population of human subjects with better therapeutic outcomes in response to the combination therapy, where a composite biomarker score equal to or lower than the control composite biomarker score is predictive that the human subject is responsive to the combination therapy comprising lenvatinib or a pharmaceutically acceptable salt thereof and everolimus.

In one embodiment, the score for each protein is a binary value based on the baseline expression level for each of the proteins. In one embodiment, the binary value is 0 or 1.

In one embodiment, the value range that is correlated with a population of human subjects with better therapeutic outcomes is determined by comparing the base line expression level with a control expression level for each of the proteins.

In one embodiment, the at least five proteins comprises HGF, MIG, IL-18BP, IL-18 and ANG-2. In another embodiment, the at least five proteins comprises TIMP-1, M-CSF, IL-18BP, ANG-2 and VEGF-A. In yet another embodiment, the at least five proteins consists of HGF, MIG, IL-18BP, IL-18 and ANG-2. In still another embodiment, the at least five proteins consists of TIMP-1, M-CSF, IL-18BP, ANG-2 and VEGF-A.

In one embodiment, the at least two proteins comprises IL-18BP and ANG-2. In another embodiment, the at least two proteins consists of IL-18BP and ANG-2.

The following embodiments are envisaged for all of the above aspects.

In one embodiment the lenvatinib or a pharmaceutically acceptable salt thereof is lenvatinib mesylate.

In one embodiment, the renal cell carcinoma is an advanced renal cell carcinoma or a metastatic renal cell carcinoma.

In some embodiments, the biological sample is selected from the group consisting of a blood sample, a serum sample, a plasma sample, a renal cell carcinoma archived tumor sample, and a renal cell carcinoma biopsy sample.

In some embodiments, the control or the control expression level is a pre-established cut-off value. In one embodiment, the pre-established cut-off value is an expression level of a protein that is determined based on receiver operating characteristic (ROC) analysis or percentile analysis predicting tumor response with a higher positive predictive value compared to no cut-off, and wherein an expression level of a protein equal to or higher than the pre-established cut-off value is a high expression level and a value lower than the pre-established cut-off value is a low expression level. The tumor response is an objective response rate (ORR), a clinical benefit rate (CBR), or % of maximum tumor shrinkage. In another embodiment, the pre-established cut-off value is an expression level of a protein that is determined based on simulation models or percentile analysis predicting survival, and wherein an expression level of a protein equal to or higher than the pre-established cut-off value is a high expression level of a protein and a value lower than the pre-established cut-off value is a low expression level of a protein. In this context, survival is progression free survival (PFS) or overall survival (OS). In some embodiments, ORR, CBR, or PFS and OS are defined by RECIST 1.1 Response Criteria, set forth in Eisenhauer, E.A. et al., Eur. J. Cancer 45:228-247 (2009).

In some embodiments, the control composite biomarker score is a pre-established cut-off value. In one embodiment, the pre-established cut-off value is a composite biomarker score wherein a score for each protein is determined based on ROC analysis or percentile analysis predicting tumor response with a higher positive predictive value compared to no cut-off, and wherein a composite biomarker score of equal to or higher than the pre-established cut-off value is a high composite biomarker score and a composite biomarker score lower than the pre-established cut-off value is a low composite biomarker score. The tumor response is an ORR, a CBR, or % of maximum tumor shrinkage. In another embodiment, the pre-established cut-off value is a composite biomarker score that is determined based on simulation models or percentile analysis predicting survival, and wherein a composite biomarker score equal to or higher than the pre-established cut-off value is a high composite biomarker score and a composite biomarker score lower than the pre-established cut-off value is a low composite biomarker score. In this context, survival is PFS or OS.

In some embodiments, the method further includes communicating the test results to the subject's health care provider. In certain embodiments, the method further includes modifying the subject's medical record to indicate that the subject is responsive to or not responsive to a combination therapy comprising lenvatinib or a pharmaceutically acceptable salt thereof and everolimus. In specific embodiments, the record is created on a computer readable medium. In certain embodiments, the method further includes prescribing a combination therapy comprising lenvatinib or a pharmaceutically acceptable salt thereof and everolimus for the subject if the baseline expression profile of at least one protein selected from the group consisting of IL-18BP, ICAM-1, FGF-21 and M-CSF or the composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   is predictive that the subject is responsive to a combination therapy comprising lenvatinib or a pharmaceutically acceptable salt thereof. In some embodiments, the method further includes administering to the subject a combination therapy comprising lenvatinib or a pharmaceutically acceptable salt thereof and everolimus if the baseline expression profile of at least one protein selected from the group consisting of IL-18BP, ICAM-1, FGF-21 and M-CSF or the composite biomarker score calculated based on the baseline expression levels of:
   (A) at least five proteins comprising (i) HGF, MIG, IL-18BP, IL-18 and ANG-2, or (ii) TIMP-1, M-CSF, IL-18BP, ANG-2 and VEGF-A, or
   (B) at least two proteins comprising IL-18BP and ANG-2
      is predictive that the subject is responsive to a therapy comprising lenvatinib or a pharmaceutically acceptable salt thereof and everolimus.

In one embodiment, the expression level of a protein is determined by measuring the amount of the protein. In one embodiment, the amount of the protein can be measured by an immunological method. In some embodiments, the immunological method is selected from the group consisting of enzyme immunoassay, radioimmunoassay, chemiluminescent immunoassay, electrochemiluminescence immunoassay, latex turbidimetric immunoassay, latex photometric immunoassay, immuno-chromatographic assay, and western blotting. In another embodiment, the amount of the protein is measured by enzyme immunoassay.

In a sixth aspect, this disclosure provides lenvatinib or a pharmaceutically acceptable salt thereof for concomitant use with everolimus in treating a renal cell carcinoma in a human subject, wherein the human subject is identified by the methods described above as a subject that is responsive to a combination therapy comprising lenvatinib or a pharmaceutically acceptable salt thereof and everolimus. In some embodiments, the pharmaceutically acceptable salt of lenvatinib is lenvatinib mesylate. In one embodiment, the renal cell carcinoma is an advanced or metastatic renal cell carcinoma.

In a seventh aspect, the disclosure provides a protein detection agent for use in predicting that a human subject having, suspected of having, or at risk of developing, a renal cell carcinoma is responsive to a combination therapy comprising lenvatinib or a pharmaceutically acceptable salt thereof and everolimus. In one embodiment, the protein detection agent is an antibody binding to the protein. In some embodiments, the pharmaceutically acceptable salt of lenvatinib is lenvatinib mesylate.

In an eighth aspect, the disclosure features a kit comprising a protein detection agent for use in predicting that a human subject having, suspected of having, or at risk of developing, a renal cell carcinoma is responsive to a combination therapy comprising lenvatinib or a pharmaceutically acceptable salt thereof and everolimus. In certain embodiments, the protein detection agent is an antibody binding to the protein. In certain embodiments, the antibody is a monoclonal antibody. In other embodiments, the antibody is a polyclonal antibody. In certain embodiments, the antibody is conjugated with a detectable agent. In one embodiment, the detectable agent is horse radish peroxidase, biotin, a fluorescent moiety, a radioactive moiety, a histidine tag, or a peptide tag. In one embodiment, the detectably labeled antibody is coated on a microplate. In certain embodiments, the microplate is a 96 well microplate. In certain embodiments, the kit optionally includes one or more concentration standards, one or more buffers (e.g., wash buffers), one or more diluents (e.g., assay and/or calibration diluents), and one or more reagents that facilitate detecting whether the protein detection agent specifically binds the protein in a biological sample obtained from the subject (e.g., color reagents, stop solutions). In some embodiments, the pharmaceutically acceptable salt of lenvatinib is lenvatinib mesylate.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. In case of conflict, the present application, including definitions, will control.

Other features and advantages of the invention will be apparent from the following detailed description, and from the claims.

### [Brief Description of Drawings]

[Fig. 1]Fig. 1 is Kaplan Meier(K-M) plots of PFS by CBS Category (0-2 vs. 3-5) in the LEN/EVE and EVE Arms in the CBS analysis (5 biomarkers).
[Fig. 2]Fig. 2 is K-M plots of OS by CBS Category (0-2 vs. 3-5) in the LEN/EVE and EVE Arms in the CBS analysis (5 biomarkers).
[Fig. 3]Fig. 3 is K-M plots of PFS by CBS Category (0 vs. 1-2) in the LEN/EVE and EVE Arms in the CBS analysis (2 biomarkers).
[Fig. 4]Fig. 4 is K-M plots of OS by CBS Category (0 vs. 1-2) in the LEN/EVE and EVE Arms in the CBS analysis (2 biomarkers).

### [Description of Embodiments]

This disclosure provides methods and compositions for identifying a renal cell carcinoma subject (such as a human patient) who is responsive to a combination therapy comprising lenvatinib or a pharmaceutically acceptable salt thereof (e.g., lenvatinib mesylate) and everolimus. The disclosure provides a baseline expression level of at least one protein selected from the group consisting of IL-18BP, ICAM-1, FGF-21 and M-CSF, or a composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   as a predictive biomarker to identify those subjects having, suspected of having, or at risk of developing, renal cell carcinoma (e.g., advanced or metastatic renal cell carcinoma) for whom administering a combination therapy comprising lenvatinib or a pharmaceutically acceptable salt thereof (e.g., lenvatinib mesylate) and everolimus is recommended. The biomarkers including the composite biomarker score, compositions, and methods described herein are useful in selecting appropriate therapeutic modalities (e.g., combination therapy comprising lenvatinib or a pharmaceutically acceptable salt thereof (e.g., lenvatinib mesylate) and everolimus or an alternative renal cell carcinoma therapy) for subjects suffering from, suspected of having or at risk of developing renal cell carcinoma. Furthermore, this application provides methods of selecting patients having, suspected of having, or at risk of developing, renal cell carcinoma that could benefit from a combination therapy comprising lenvatinib or a pharmaceutically acceptable salt thereof (e.g., lenvatinib mesylate) and everolimus as well as methods of treatment.

### Definitions

The term "subject" means a mammal, including but not limited to, a human, a chimpanzee, an orangutan, a gorilla, a baboon, a monkey, a mouse, a rat, a pig, a horse, a dog, and a cow.

The term "combination therapy" means a therapy that concomitantly administers two or more drugs to a patient. The two or more drugs can be administered simultaneously, substantially simultaneously, or sequentially. In some cases, the two or more drugs may be formulated together (e.g., into a single tablet or capsule). In other cases, the two or more drugs are not co- formulated (e.g., they are administered as separate tablets or capsules).

The term "lenvatinib" refers to 4-(3-chloro-4-(cyclopropylaminocarbonyl)aminophenoxy)-7-methoxy-6-quinolinecarb oxamide. This compound is disclosed in Example 368 (see, column 270) of U.S. Patent No. 7,253,286. The term "lenvatinib compound" refers to "lenvatinib or a pharmaceutically acceptable salt thereof." An example of a pharmaceutically acceptable salt of lenvatinib is lenvatinib mesylate. Lenvatinib mesylate is also referred to as E7080. Lenvatinib mesylate has been approved as LENVIMA^{(Registered Trademark)} by the U.S. Food and Drug Administration for the treatment of patients with locally recurrent or metastatic, progressive, radioactive iodine-refractory differentiated thyroid cancer, unresectable hepatocellular carcinoma, or in combination with everolimus, for the treatment of patients with advanced renal cell carcinoma.

The term "pharmaceutically acceptable salt" is not particularly restricted as to the type of salt. Examples of such salts include, but are not limited to, inorganic acid addition salt such as hydrochloric acid salt, sulfuric acid salt, carbonic acid salt, bicarbonate salt, hydrobromic acid salt and hydriodic acid salt; organic carboxylic acid addition salt such as acetic acid salt, maleic acid salt, lactic acid salt, tartaric acid salt and trifluoroacetic acid salt; organic sulfonic acid addition salt such as methanesulfonic acid salt, hydroxymethanesulfonic acid salt, hydroxyethanesulfonic acid salt, benzenesulfonic acid salt, toluenesulfonic acid salt and taurine salt; amine addition salt such as trimethylamine salt, triethylamine salt, pyridine salt, procaine salt, picoline salt, dicyclohexylamine salt, N,N'-dibenzylethylenediamine salt, N-methylglucamine salt, diethanolamine salt, triethanolamine salt, tris(hydroxymethylamino)methane salt and phenethylbenzylamine salt; and amino acid addition salt such as arginine salt, lysine salt, serine salt, glycine salt, aspartic acid salt and glutamic acid salt. In one embodiment, the pharmaceutically acceptable salt is a methanesulfonic acid salt ("mesylate"). The methanesulfonic acid salt form (i.e., the mesylate) of 4-(3-chloro-4-(cyclopropylaminocarbonyl)aminophenoxy)-7-methoxy-6-quinolinecarb-oxamide is disclosed in US Patent 7,612,208.

The term "everolimus" refers to (1*R*,9*S*,12*S*,15*R*,16*E*,18*R*,19*R*,21*R*,23*S*,24*E*,26*E*,28*E*,30*S*,32*S*,35*R*)-1,18-dihydroxy-12- {(1*R*)-2-[(1*S*,3*R*,4*R*)-4-(2-hydroxyethoxy)-3-methoxycyclohexyl] -1 -methyl ethyl} - 19,30-dimethoxy-15,17,21,23,29,35-hexamethyl-11,36-dioxa-4-aza-tricyclo[30.3.1.0^{4,9} ]hexatriaconta-16,24,26,28-tetraene-2,3,10,14,20-pentaone. This compound is disclosed in U.S. Patent No. 5,665,772. Everolimus has been approved as AFINITOR ^{(Registered Trademark)} by U.S. Food and Drug Administration for the treatment of various diseases, including advanced renal cell carcinoma after failure of treatment with sunitinib or sorafenib.

The term "protein" means any peptide-linked chain of amino acids, regardless of length or post-translational modification. Typically, a protein described herein is "isolated" when it constitutes at least 60%, by weight, of the total protein in a preparation, e.g., 60% of the total protein in a sample. In some embodiments, a protein described herein consists of at least 75%, at least 90%, or at least 99%, by weight, of the total protein in a preparation.

The term "VEGF-targeted therapy" means any therapy comprising an administration of an anti-tumor agent that acts on vascular endothelial growth factor (VEGF) receptor(s). Examples of anti-tumor agents used for VEGF-targeted therapy are: sunitinib, sorafenib, pazopanib, bevacizumab, axitinib, vatalanib and tivozanib.

The term "responds/responsive to a therapy" means that the subject administered with the therapy shows a positive response to the therapy provided.
Examples of such a positive response are: a decrease in tumor size, a decrease in metastasis of a tumor, or an increased period of survival after treatment. To a subject who is predicted as responsive to a combination therapy, the combination therapy is recommendable as a preferable treatment. Examples of situation where a combination therapy is recommendable as a preferable treatment are: a combination therapy comprising two particular drugs is predicted as more effective than other combination therapy(s) comprising two drugs at least one of which are different from said two particular drugs; a combination therapy comprising two particular drugs is predicted as more effective than monotherapy with one of, or each of, said two drugs. In one embodiment, the situation is where a combination therapy lenvatinib or a pharmaceutically acceptable salt thereof (e.g., lenvatinib mesylate) and everolimus is predicted as more effective than monotherapy with everolimus, both of which are approved by the U.S. Food and Drug Administration as a therapy for advanced renal cell carcinoma.

The term "baseline level" of a protein in a sample from a subject means the amount of that protein in the sample before administration of the subject with a combination therapy of everolimus and lenvatinib or a pharmaceutically acceptable salt thereof.

The term "single biomarker analysis" means an analysis in which a determination for identification, prediction or selection of patient's responsiveness to a treatment is made by a single biomarker expression level. When more than one biomarkers are analyzed, the determination is made for each protein. More specifically, the single biomarker analysis of the present disclosure is an analysis using at least one protein selected from the group consisting of IL-18BP, ICAM-1, FGF-21 and M-CSF in a biological sample to identify or predict if a human subject having, suspected of having, or at risk of developing, a renal cell carcinoma is responsive to a combination therapy comprising lenvatinib or a pharmaceutically acceptable salt thereof (e.g., lenvatinib mesylate) and everolimus disclosed herein.

The term "composite biomarker score analysis" means an analysis in which a determination for identification, prediction or selection of patient's responsiveness to a treatment is made by an integrated evaluation of expression levels of multiple proteins. The proteins used in the composite biomarker score analysis can be selected from proteins which have associations with the desired therapeutic outcome. More specifically, the composite biomarker score analysis in the present disclosure is an analysis using composite marker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   to identify or predict whether a human subject having, suspected of having, or at risk of developing, a renal cell carcinoma is responsive to a combination therapy comprising lenvatinib or a pharmaceutically acceptable salt thereof (e.g., lenvatinib mesylate) and everolimus disclosed herein. The composite biomarker score can be calculated by the steps of:
   measuring or having measured baseline expression levels of:
   (A) at least five proteins comprising (i) HGF, MIG, IL-18BP, IL-18 and ANG-2, or (ii) TIMP-1, M-CSF, IL-18BP, ANG-2 and VEGF-A, or
   (B) at least two proteins comprising IL-18BP and ANG-2
      in the biological sample;
      determining or having determined a score for each protein based on its baseline expression level; and
      summing up or having summed up the scores to obtain a composite biomarker score.

A medical practitioner can perform the measurement of the baseline expression levels, the score determination or the calculation of the composite biomarker score by himself, or can have the other practitioners or health care providers do.

In one embodiment, a higher score can be assigned to each protein if a baseline expression level falls within the value range which is correlated with a population of human subjects with better therapeutic outcome. In such a case, a composite biomarker score equal to or higher than a control composite biomarker score is predictive that the human subject is responsive to the combination therapy comprising lenvatinib or a pharmaceutically acceptable salt thereof and everolimus. In another embodiment, a lower score can be assigned to each protein if a baseline expression level falls within the value range which is correlated with a population of human subjects with better therapeutic outcome. In such a case, a composite biomarker score equal to or lower than a control composite biomarker score is predictive that the human subject is responsive to the combination therapy comprising lenvatinib or a pharmaceutically acceptable salt thereof and everolimus.

The score based on its baseline expression level in each of the proteins can be a binary value. The binary value can be any value. The binary value can be selected from zero and positive numbers. The binary value can also be selected from zero and positive integers. One example of the binary value is 0 or 1. The value range which is correlated with a population of human subjects with better therapeutic outcome can be determined by comparing the baseline expression level with a control expression level for each of the proteins. For HGF, MIG, IL-18BP, IL-18, ANG-2, TIMP-1, M-CSF, and VEGF-A, the value range which is correlated with a population of human subjects with better therapeutic outcome can be the value range which is lower than the control expression level for each protein. The proteins used in the composite biomarker score analysis are selected from proteins which have associations (e.g. strong associations or correlations) with the desired therapeutic outcome. The proteins which have associations with the desired therapeutic outcome can be determined by the statistical methods known in the art. For example, dichotomized analysis with median cutoff point using univariate Cox regression analysis and log-rank test can be conducted to identify candidate biomarkers which have associations with the desired therapeutic outcome and several candidate biomarkers with strongest association can be selected for the composite biomarker score analysis. The "at least five proteins" can be more than five proteins comprising (i) HGF, MIG, IL-18BP, IL-18 and ANG-2, or (ii) TIMP-1, M-CSF, IL-18BP, ANG-2 and VEGF-A, or five proteins of (i) HGF, MIG, IL-18BP, IL-18 and ANG-2, or (ii) TIMP-1, M-CSF, IL-18BP, ANG-2 and VEGF-A. The "at least two proteins" can be more than two proteins comprising IL-18BP and ANG-2, or two proteins of IL-18BP and ANG-2. One method of composite biomarker score analysis is described in Voss, et al., Br J Cancer. 2016 Mar 15;114(6):642-9.

A low baseline expression level (e.g., protein or mRNA expression) compared to a control of at least one protein selected from the group consisting of IL-18BP, ICAM-1, FGF-21 and M-CSF is indicative/predictive that a subject is responsive to a combination therapy comprising a lenvatinib compound (e.g., lenvatinib mesylate) and everolimus. For example, low baseline expression levels (compared to a control) of at least one protein selected from the group consisting of IL-18BP, ICAM-1, FGF-21 and M-CSF in a biological sample obtained from a subject prior to treatment with the therapy are predictive that the subject is responsive to a combination therapy comprising a lenvatinib compound (e.g., lenvatinib mesylate) and everolimus.

A high composite marker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2,
   compared to a control composite biomarker score, is indicative/predictive that a subject is responsive to a combination therapy comprising a lenvatinib compound (e.g., lenvatinib mesylate) and everolimus, when a larger value is assigned as a score for each protein value if a baseline expression level falls within the value range which is correlated with a population of human subjects with better therapeutic outcome. For example, a high composite marker score calculated based on the baseline expression levels of:
   (A) at least five proteins comprising (i) HGF, MIG, IL-18BP, IL-18 and ANG-2, or (ii) TIMP-1, M-CSF, IL-18BP, ANG-2 and VEGF-A, or
   (B) at least two proteins comprising IL-18BP and ANG-2,
      in a biological sample obtained from a subject prior to treatment with the therapy, compared to a control composite marker score, is predictive that the subject is responsive to a combination therapy comprising a lenvatinib compound (e.g., lenvatinib mesylate) and everolimus.

A low composite marker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2,
   compared to a control composite biomarker score, is indicative/predictive that a subject is responsive to a combination therapy comprising a lenvatinib compound (e.g., lenvatinib mesylate) and everolimus, when a smaller value is assigned as a score for each protein value if a baseline expression level falls within the value range which is correlated with a population of human subjects with better therapeutic outcome. For example, a low composite marker score calculated based on the baseline expression levels of:
   (A) at least five proteins comprising (i) HGF, MIG, IL-18BP, IL-18 and ANG-2, or (ii) TIMP-1, M-CSF, IL-18BP, ANG-2 and VEGF-A, or
   (B) at least two proteins comprising IL-18BP and ANG-2,
      in a biological sample obtained from a subject prior to treatment with the therapy, compared to a control composite marker score, is predictive that the subject is responsive to a combination therapy comprising a lenvatinib compound (e.g., lenvatinib mesylate) and everolimus.

In certain embodiments, a subject is determined to respond to a combination therapy comprising a lenvatinib compound (e.g., lenvatinib mesylate) and everolimus, if the subject shows a partial response following treatment with the therapy. "Partial Response" means at least 30% decrease in the sum of the longest diameter (LD) of target lesions, taking as reference the baseline summed LD. In some embodiments, a subject is determined to respond to a combination therapy comprising a lenvatinib compound and everolimus, if the subject shows tumor shrinkage post-treatment with the therapy. "% of maximum tumor shrinkage" (MTS) means percent change of sum of diameters of target lesions, taking as reference the baseline sum diameters. In other embodiments, a subject is determined to respond to a combination therapy comprising a lenvatinib compound and everolimus, if the subject shows overall survival. "Overall Survival" (OS) refers to the time from randomization until death from any cause. "Randomization" means randomization of a patient into a test group or a control group when therapy plan for a patient is determined. In some embodiments, a subject is determined to respond to a combination therapy comprising a lenvatinib compound and everolimus, if the subject shows both overall survival and tumor shrinkage. In other embodiments, a subject is determined to respond to a combination therapy comprising a lenvatinib compound and everolimus, if the subject shows progression free survival. "Progression Free Survival" (PFS) refers to the time from the date of randomization to the date of first documentation of disease progression or death, whichever occurs first. In some embodiments, a subject is determined to respond to a combination therapy comprising a lenvatinib compound and everolimus, if the subject shows both progression free survival and tumor shrinkage.

This disclosure provides methods of identifying a subject having renal cell carcinoma who is more likely to have survival benefits (e.g., OS) following a combination therapy comprising a lenvatinib compound (e.g., lenvatinib mesylate) and everolimus than a monotherapy comprising everolimus only. In this method, a biological sample of the subject, obtained prior to treatment with the combination therapy comprising a lenvatinib compound (e.g., lenvatinib mesylate) and everolimus, is assayed and the expression level of at least one protein selected from the group consisting of IL-18BP, ICAM-1, FGF-21 and M-CSF, or the expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2, is/are measured. A lower baseline expression of at least one protein selected from the group consisting of IL-18BP, ICAM-1, FGF-21 and M-CSF compared to a control indicates that the subject will more likely have survival benefits (e.g., OS) following combination therapy comprising a lenvatinib compound (e.g., lenvatinib mesylate) and everolimus than a monotherapy comprising everolimus only. A higher composite marker score calculated based on the baseline expression levels of:
   (A) at least five proteins comprising (i) HGF, MIG, IL-18BP, IL-18 and ANG-2, or (ii) TIMP-1, M-CSF, IL-18BP, ANG-2 and VEGF-A, or
   (B) at least two proteins comprising IL-18BP and ANG-2,
      compared to a control composite biomarker score indicates that the subject will more likely have survival benefits following combination therapy comprising a lenvatinib compound (e.g., lenvatinib mesylate) and everolimus than a monotherapy comprising everolimus only, in the case that a larger value is assigned as a score for each protein if a baseline expression level falls within the value range. A lower composite marker score calculated based on the baseline expression levels of:
      (A) at least five proteins comprising (i) HGF, MIG, IL-18BP, IL-18 and ANG-2, or (ii) TIMP-1, M-CSF, IL-18BP, ANG-2 and VEGF-A, or
      (B) at least two proteins comprising IL-18BP and ANG-2,
         compared to a control composite biomarker score indicates that the subject will more likely have survival benefits (e.g., OS) following combination therapy comprising a lenvatinib compound (e.g., lenvatinib mesylate) and everolimus than a monotherapy comprising everolimus only, in the case that a smaller value is assigned as a score for each protein if a baseline expression level falls within the value range.

In certain embodiments, a lenvatinib compound (e.g., lenvatinib mesylate) can be administered to a subject of the present invention orally once daily at a dosage of 12, 18 or 24 mg (each calculated as lenvatinib free base).

In certain embodiments, everolimus can be administered to a subject of the present invention orally once daily at a dosage of 5 or 10 mg.

In certain embodiments, a lenvatinib compound (e.g., lenvatinib mesylate) can be administered to a subject of the present invention at a dosage of 18 mg orally once daily with everolimus 5 mg orally once daily. In certain embodiments, a lenvatinib compound (e.g., lenvatinib mesylate) can be administered to a subject of the present invention at a dosage of 12 mg orally once daily with everolimus 5 mg orally once daily. In certain embodiments, a lenvatinib compound (e.g., lenvatinib mesylate) can be administered to a subject of the present invention at a dosage of 18 mg orally once daily with everolimus 10 mg orally once daily. In certain embodiments, a lenvatinib compound (e.g., lenvatinib mesylate) can be administered to a subject of the present invention at a dosage of 12 mg orally once daily with everolimus 10 mg orally once daily. In certain embodiments, a lenvatinib compound (e.g., lenvatinib mesylate) can be administered to a subject of the present invention at a dosage of 24 mg orally once daily with everolimus 10 mg orally once daily.

The amount of a protein can be measured using any method known in the art such as an immunological assay. Examples of such methods include enzyme immunoassay, radioimmunoassay, chemiluminescent immunoassay, electrochemiluminescence immunoassay, latex turbidimetric immunoassay, latex photometric immunoassay, immuno-chromatographic assay, and western blotting. In certain embodiments, the amount of a protein is measured by enzyme immunoassay.

### Controls

As described above, the methods of the present invention can involve, measuring or having measured a baseline expression level of a protein in a biological sample from a subject having, suspected of having or at risk of developing renal cell carcinoma, wherein the expression level of the protein, compared to a control, predicts that the subject is responsive to (or benefit from) a combination treatment comprising a lenvatinib compound (e.g., lenvatinib mesylate) and everolimus or expression levels of the proteins, compared to a control, is used to calculate a composite marker score. In certain embodiments, when a baseline expression level of at least one protein selected from the group consisting of IL-18BP, ICAM-1, FGF-21 and M-CSF in a biological sample from a subject having, suspected of having or at risk of developing renal cell carcinoma is lower than the control, the subject is identified as responsive to a combination therapy comprising a lenvatinib compound and everolimus. In certain embodiments using a composite biomarker score analysis, when a baseline expression level for each protein of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   falls within a value range which is correlated with a population of human subjects with better therapeutic outcome, compared to the control, a certain score is assigned. In one embodiment, a larger value can be assigned as a score for each protein if a baseline expression level falls within the value range which is correlated with a population of human subjects with better therapeutic outcome. In another embodiment, a smaller value can be assigned as a score for each protein if a baseline expression level falls within the value range which is correlated with a population of human subjects with better therapeutic outcome.

In this context, the term "control" includes a sample (e.g., from the same tissue) obtained from a subject who is not responsive to a combination therapy comprising a lenvatinib compound (e.g., lenvatinib mesylate) and everolimus. Such subject who is not responsive to a combination therapy comprising a lenvatinib compound (e.g., lenvatinib mesylate) and everolimus may include a subject who is predicted to respond to the combination therapy but such response to the combination therapy is not significantly better than a predicted response to a monotherapy with everolimus. The term "control" also includes a sample (e.g., from the same tissue) obtained in the past from a subject who is known to be not responsive to a combination therapy comprising a lenvatinib compound and everolimus and used as a reference for future comparisons to test samples taken from subjects for which necessity for the combination therapy is to be predicted.

In some embodiments, a "positive control" may be used instead of a "control." The "positive control" expression level in a particular cell type or tissue may alternatively be pre-established by an analysis of one or more subjects that have been identified as responsive to a combination therapy comprising lenvatinib compound (e.g., lenvatinib mesylate) and everolimus. This pre-established reference value (which may be an average or median expression level taken from multiple subjects that have been identified as responsive to a combination therapy) may then be used as the "positive control" expression level in the comparison with the test sample. In such a comparison, the subject is predicted to be responsive to a combination therapy comprising a lenvatinib compound (e.g., lenvatinib mesylate) and everolimus if the expression level of at least one protein selected from the group consisting of IL-18BP, ICAM-1, FGF-21 and M-CSF being analyzed is the same as, or comparable to, the pre-established positive control reference. In an embodiments applying a composite biomarker score analysis, if a baseline expression level of each biomarker of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2,
   being the same as, or comparable to the pre-established positive control reference, a certain score is assigned as the baseline expression level falls within the value range which is correlated with a population of human subjects with better therapeutic outcome. In some embodiments, a larger value can be assigned as a score for each protein if a baseline expression level falls within the value range which is correlated with a population of human subjects with better therapeutic outcome. In other embodiments, a smaller value can be assigned as a score for each protein if a baseline expression level falls within the value range which is correlated with a population of human subjects with better therapeutic outcome.

In certain embodiments, the "control" is a pre-determined cut-off value.

### Control Biomarker Score

As described above, the methods of the present invention using a composite biomarker score analysis can involve determining a composite biomarker score by summing up scores each of which is determined based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2,
   in a biological sample from a subject having, suspected of having or at risk of developing renal cell carcinoma, wherein the composite biomarker score, compared to a control composite biomarker score, predicts that the subject is responsive to (or benefit from) a combination treatment comprising a lenvatinib compound (e.g., lenvatinib mesylate) and everolimus. In certain embodiments, when a larger value is assigned as a score for each protein if a baseline expression level falls within the value range which is correlated with a population of human subjects with better therapeutic outcome and a composite biomarker score is equal to or higher than the control, the subject is identified as responsive to a combination therapy comprising a lenvatinib compound and everolimus. In certain embodiments, when a smaller value is assigned as a score for each protein if a baseline expression level falls within the value range which is correlated with a population of human subjects with better therapeutic outcome and a composite biomarker score is equal to or lower than the control, the subject is identified as responsive to a combination therapy comprising a lenvatinib compound and everolimus. In this context, the term "control composite biomarker score" includes a composite biomarker score from a sample (e.g., from the same tissue) obtained from a subject who is not responsive to a combination therapy comprising a lenvatinib compound (e.g., lenvatinib mesylate) and everolimus. Such subject who is not responsive to a combination therapy comprising a lenvatinib compound (e.g., lenvatinib mesylate) and everolimus may include a subject who is predicted to respond to the combination therapy but such response to the combination therapy is not significantly better than a predicted response to a monotherapy with everolimus. The term "control composite biomarker score" also includes a composite biomarker score from a sample (e.g., from the same tissue) obtained in the past from a subject who is known to be not responsive to a combination therapy comprising a lenvatinib compound and everolimus and used as a reference for future comparisons to test samples taken from subjects for which responsiveness to the combination therapy is to be predicted.

In some embodiments, a "positive control composite biomarker score" may be used instead of a "control composite biomarker score". The "positive control composite biomarker score" from a particular cell type or tissue may alternatively be pre-established by an analysis of one or more subjects that have been identified as responsive to a combination therapy comprising lenvatinib compound (e.g., lenvatinib mesylate) and everolimus. This pre-established reference value (which may be an average or median composite biomarker score taken from multiple subjects that have been identified as responsive to a combination therapy) may then be used as the "positive control composite biomarker score" in the comparison with the test sample. In such a comparison, the subject is predicted to be responsive to a combination therapy comprising a lenvatinib compound (e.g., lenvatinib mesylate) and everolimus if the composite biomarker score calculated based on the expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2,
   being analyzed is the same as, or comparable to, the pre-established reference positive control composite biomarker score.

In certain embodiments, the "control composite biomarker score" is a pre-determined cut-off value.

### Cut-Off Values

In some embodiments, the methods described herein include determining if the expression levels of the protein fall above or below a predetermined cut-off value.

In accordance with the methods and compositions using a single biomarker analysis described herein, a reference baseline expression level of a protein is identified as a cut-off value, above or below of which is predictive of necessity for a combination therapy comprising a lenvatinib compound (e.g., lenvatinib mesylate) and everolimus. Further, in accordance with the method and compositions using a composite biomarker score analysis, a reference baseline expression level of a protein is identified as a cut-off value, above or below of which defines a value range which is correlated with a population of human subjects with better therapeutic outcome for each biomarker protein. Some cut-off values are not absolute in that clinical correlations can still remain significant over a range of values on either side of the cutoff; however, it is possible to select an optimal cut-off value (e.g. varying H-scores) of an expression level of a protein for a particular sample type. Cut-off values determined for use in the methods described herein can be compared with, e.g., published ranges of expression level but can be individualized to the methodology used and patient population. It is understood that improvements in optimal cut-off values could be determined depending on the sophistication of statistical methods used and on the number and source of samples used to determine reference level values for the different sample types. Therefore, established cut-off values can be adjusted up or down, on the basis of periodic re-evaluations or changes in methodology or population distribution.

The reference expression level of a protein can be determined by a variety of methods. The reference level can be determined by comparison of the expression level of the protein of interest in, e.g., populations of subjects (e.g., patients) that are responsive to a combination therapy comprising a lenvatinib compound (e.g., lenvatinib mesylate) and everolimus or not responsive to a combination therapy comprising a lenvatinib compound and everolimus. This can be accomplished, for example, by histogram analysis, in which an entire cohort of patients is graphically presented, wherein a first axis represents the expression level of the protein and a second axis represents the number of subjects in the cohort whose sample contains one or more expression levels of the protein. Determination of the reference expression levels of the protein can then be made based on an expression level which best distinguishes these separate groups. The reference level can be a single number, equally applicable to every subject, or the reference level can vary, according to specific subpopulations of subjects. For example, older subjects can have a different reference level than younger subjects for the same cancer. In addition, a subject with more advanced disease (e.g., an advanced or metastatic renal cell carcinoma) can have a different reference value than one with a milder form of the disease.

The pre-established cut-off value can be a protein expression level or a composite biomarker score that is determined based on ROC analysis. ROC curves are used to determine a cut-off value for a clinical test. Consider the situation where there are two groups of patients and by using an established standard technique one group is known to be responsive to a combination therapy comprising lenvatinib compound and everolimus, and the other is known to be not responsive to a combination therapy comprising lenvatinib compound and everolimus. A measurement using a biological sample from all members of the two groups is used to test for the necessity for a combination therapy comprising lenvatinib compound and everolimus.

In the single biomarker analysis of the present disclosure, the test will find some, but not all, subjects that are responsive to a combination therapy comprising lenvatinib compound and everolimus. The ratio of the subjects responsive to the combination therapy found by the test to the total number of the subjects responsive to the combination therapy (known by the established standard technique) is the true positive rate (also known as sensitivity). In the single biomarker analysis of the present disclosure, the test will find some, but not all, the subjects not responsive to a combination therapy comprising a lenvatinib compound and everolimus. The ratio of the subjects not responsive to the combination therapy found by the test to the total number of the subjects not responsive to the combination therapy (known by the established standard technique) is the true negative rate (also known as specificity). The hope is that the ROC curve analysis of the test above will find a cut-off value that will minimize the number of false positives and false negatives.

In the composite biomarker score analysis of the present disclosure, the test will find some, but not all, subjects whose protein expression levels fall within a value range which is correlated with a population of human subjects with better therapeutic outcome of a combination therapy comprising lenvatinib compound and everolimus. The ratio of the subjects with better therapeutic outcome found by the test to the total number of the subjects with better therapeutic outcome (known by the established standard technique) is the true positive rate (also known as sensitivity). In the composite biomarker score analysis of the present disclosure, the test will find some, but not all, subjects whose protein expression levels do not fall within a value range which is correlated with a population of human subjects with better therapeutic outcome of a combination therapy comprising lenvatinib compound and everolimus. The ratio of the subjects without better therapeutic outcome found by the test to the total number of the subjects without better therapeutic outcome by the combination therapy (known by the established standard technique) is the true negative rate (also known as specificity). The hope is that the ROC curve analysis of the test above will find a cut-off value that will minimize the number of false positives and false negatives.

In the composite biomarker score analysis of the present disclosure, the test will find some, but not all, subjects that are responsive to a combination therapy comprising lenvatinib compound and everolimus. The ratio of the subjects responsive to the combination therapy found by the test to the total number of the subjects responsive to the combination therapy (known by the established standard technique) is the true positive rate (also known as sensitivity). In the composite biomarker score analysis of the present disclosure, the test will find some, but not all, subjects not responsive to a combination therapy comprising a lenvatinib compound and everolimus. The ratio of the subjects not responsive to the combination therapy found by the test to the total number of the subjects not responsive to the combination therapy (known by the established standard technique) is the true negative rate (also known as specificity). The hope is that the ROC curve analysis of the test above will find a cut-off value that will minimize the number of false positives and false negatives.

A ROC is a graphical plot which illustrates the performance of a binary class stratifier system as its discrimination threshold is varied. It is created by plotting the fraction of true positives out of the positives versus the fraction of false positives out of the negatives, at various threshold settings.

In one embodiment, the expression level of the protein is determined based on ROC analysis predicting tumor response with a positive predictive value, wherein the expression level of the protein equal to or higher than the pre-established cut-off value is a high expression levels of the protein and a value lower than the pre-established cut-off value is a low expression level of the protein. The positive predictive value is the proportion of positive test results that are true positives; it reflects the probability that a positive test reflects the underlying condition being tested for. Methods of constructing ROC curves and determining positive predictive values are well known in the art. In certain embodiments, tumor response is ORR, CBR or % of maximum tumor shrinkage.

In a similar manner, the composite biomarker score is determined based on ROC analysis.

In another embodiment, the pre-established cut-off value can be an expression levels of a protein that is determined based on simulation models predicting survival, wherein an expression levels of the protein equal to or higher than the pre-established cut-off value is a high expression levels of the protein and a value lower than the pre-established cut-off value is a low expression levels of the protein. In some embodiments, the survival is PFS. In other embodiments, the survival is OS.

In another embodiment, the pre-established cut-off value can be a composite biomarker score that is determined based on simulation models predicting survival, wherein a composite biomarker score equal to or higher than the pre-established cut-off value is a high composite biomarker score and a value lower than the pre-established cut-off value is a low composite biomarker score. In some embodiments, the survival is PFS. In other embodiments, the survival is OS.

In certain embodiments, the pre-established cut-off value is within a range of 20th percentile to 80th percentile of populations of subjects. In some embodiments, the pre-established cut-off value is within a range of 20th percentile to 75th percentile, 25th percentile to 80th percentile, or 25th percentile to 75th percentile of populations of subjects. In some embodiments, the pre-established cut-off value is median, first tertile, second tertile, first quantile, third quantile, first quintile, second quintile, third quintile, or forth quintile of populations of subjects.

### Biological Samples

Suitable biological samples for the methods described herein include any biological fluid, cell, tissue, or fraction thereof, which contains proteins to be measured. A biological sample can be, for example, a specimen obtained from a human subject or can be derived from such a subject. For example, a sample can be a tissue section obtained by biopsy, archived tumor tissue, or cells that are placed in or adapted to tissue culture. A biological sample can also be a biological fluid such as blood, plasma, serum, or such a sample absorbed onto a substrate (e.g., glass, polymer, paper). A biological sample can also include a renal cell carcinoma tissue sample. In specific embodiments, the biological sample is a tumor cell(s) or a tumor tissue obtained from a region of the subject suspected of containing a tumor or a pre-cancerous lesion. For example, the biological sample may be a renal cell carcinoma tumor sample. A biological sample can be further fractionated, if desired, to a fraction containing particular cell types. For example, a blood sample can be fractionated into serum or into fractions containing particular types of blood cells such as red blood cells or white blood cells (leukocytes). If desired, a sample can be a combination of samples from a subject such as a combination of a tissue and fluid sample.

The biological samples can be obtained from a subject having, suspected of having, or at risk of developing, a renal cell carcinoma. In certain embodiments, the subject has advanced or metastatic renal cell carcinoma. In some embodiments, the subject has recurrent renal cell carcinoma. In other embodiments, the subject has an unresectable advanced or metastatic renal cell carcinoma. In other embodiments, the subject has a stage III renal cell carcinoma. In certain embodiments, the subject has a stage IV renal cell carcinoma.

Any suitable methods for obtaining the biological samples can be employed, although exemplary methods include, e.g., phlebotomy, fine needle aspirate biopsy procedure. Samples can also be collected, e.g., by microdissection (e.g., laser capture microdissection (LCM) or laser microdissection (LMD)).

Methods for obtaining and/or storing samples that preserve the activity or integrity of molecules (e.g., nucleic acids or proteins) in the sample are well known to those skilled in the art. For example, a biological sample can be further contacted with one or more additional agents such as buffers and/or inhibitors, including one or more of nuclease, protease, and phosphatase inhibitors, which preserve or minimize changes in the molecules (e.g., nucleic acids or proteins) in the sample. Such inhibitors include, for example, chelators such as ethylenediamine tetraacetic acid (EDTA), ethylene glycol bis(P-aminoethyl ether) N,N,N1,N1-tetraacetic acid (EGTA), protease inhibitors such as phenylmethylsulfonyl fluoride (PMSF), aprotinin, leupeptin, antipain, and the like, and phosphatase inhibitors such as phosphate, sodium fluoride, vanadate, and the like. Suitable buffers and conditions for isolating molecules are well known to those skilled in the art and can be varied depending, for example, on the type of molecule in the sample to be characterized (see, for example, Ausubel et al. Current Protocols in Molecular Biology (Supplement 47), John Wiley & Sons, New York (1999); Harlow and Lane, Antibodies: A Laboratory Manual (Cold Spring Harbor Laboratory Press (1988); Harlow and Lane, Using Antibodies: A Laboratory Manual, Cold Spring Harbor Press (1999); Tietz Textbook of Clinical Chemistry, 3rd ed. Burtis and Ashwood, eds. W.B. Saunders, Philadelphia, (1999)). A sample also can be processed to eliminate or minimize the presence of interfering substances. For example, a biological sample can be fractionated or purified to remove one or more materials that are not of interest. Methods of fractionating or purifying a biological sample include, but are not limited to, chromatographic methods such as liquid chromatography, ionexchange chromatography, size-exclusion chromatography, or affinity chro matography. For use in the methods described herein, a sample can be in a variety of physical states. For example, a sample can be a liquid or solid, can be dissolved or suspended in a liquid, can be in an emulsion or gel, or can be absorbed onto a material.

### Determining Expression Levels of Proteins

Expression level of a protein can be determined by measuring the protein itself or mRNA encoding the protein.

In one embodiment, the expression of a protein can be determined by measuring amount or concentration of the protein. Methods of determining protein amount or concentration are well known in the art. A generally used method involves the use of antibodies specific for the target protein of interest. For example, methods of determining protein expression include, but are not limited to, western blot or dot blot analysis, immunohistochemistry (e.g., quantitative immunohistochemistry), immune-cytochemistry, enzyme-linked immunosorbent assay (ELISA), enzyme-linked immunosorbent spot (ELISPOT; Coligan, J. E., et al., eds. (1995) Current Protocols in Immunology. Wiley, New York), radioimmunoassay, chemiluminescent immunoassay, electrochemiluminescence immunoassay, latex turbidimetric immunoassay, latex photometric immunoassay, immuno-chromatographic assay, and antibody array analysis (see, e.g., U.S. Publication Nos. 20030013208 and 2004171068). Further description of many of the methods above and additional methods for detecting protein expression can be found in, e.g., Sambrook et al. (supra).

In one example, the expression level of a protein can be determined using a western blotting technique. For example, a lysate can be prepared from a biological sample, or the biological sample itself, can be contacted with Laemmli buffer and subjected to sodium-dodecyl sulfate polyacrylamide gel electrophoresis (SDS-PAGE). SDS-PAGE-resolved proteins, separated by size, can then be transferred to a filter membrane (e.g., nitrocellulose) and subjected to immunoblotting techniques using a detectably-labeled antibody specific to the protein. The amount of bound detectably-labeled antibody indicates the amount of protein in the biological sample.

In another example, an immunoassay can be used for measuring a protein expression level. As above, an immunoassay can be performed with an antibody that bears a detection moiety (e.g., a fluorescent agent or enzyme). Proteins from a biological sample can be conjugated directly to a solid-phase matrix (e.g., a multi-well assay plate, nitrocellulose, agarose, sepharose, encoded particles, or magnetic beads) or it can be conjugated to a first member of a specific binding pair (e.g., biotin or streptavidin) that attaches to a solid-phase matrix upon binding to a second member of the specific binding pair (e.g., streptavidin or biotin). Such attachment to a solid-phase matrix allows the proteins to be purified away from other interfering or irrelevant components of the biological sample prior to contact with the detection antibody and also allows for subsequent washing of unbound antibody. Here as above, the amount of bound detectably-labeled antibody indicates the amount of protein in the biological sample.

There is no particular restriction as to the form of the antibody and the present disclosure includes polyclonal antibodies, as well as monoclonal antibodies. The antiserum obtained by immunizing animals, such as rabbits with a protein or fragment thereof, as well polyclonal and monoclonal antibodies of all classes, human antibodies, and humanized antibodies produced by genetic recombination, are also included.

An intact protein or its partial peptide may be used as the antigen for immunization. As partial peptides of the proteins, for example, the amino (N)-terminal fragment of the protein and the carboxy (C)-terminal fragment can be given.

A gene encoding a protein or a fragment thereof (e.g., an immunological fragment) to be expressed is inserted into a known expression vector, and, by transforming the host cells with the vector described herein, the desired protein or a fragment thereof is recovered from outside or inside the host cells using standard methods. This protein can be used as the sensitizing antigen. Also, cells expressing the protein, cell lysates, or a chemically synthesized protein of the invention may be also used as a sensitizing antigen.

The mammal that is immunized by the sensitizing antigen is not restricted; however, it is preferable to select animals by considering the compatibility with the parent cells used in cell fusion. Generally, animals belonging to the orders rodentia, lagomorpha, or primates are used. Examples of animals belonging to the order of rodentia that may be used include, for example, mice, rats, and hamsters. Examples of animals belonging to the order of lagomorpha that may be used include, for example, rabbits. Examples of animals belonging to the order of primates that may be used include, for example, monkeys. Examples of monkeys to be used include the infraorder catarrhini (old world monkeys), for example, Macaca fascicularis, rhesus monkeys, sacred baboons, and chimpanzees.

Moreover, the antibody of the present disclosure may be an antibody fragment or modified-antibody, so long as it binds to the protein to be measured. For instance, Fab, F(ab')₂, Fv, or single chain Fv (scFv) in which the H chain Fv and the L chain Fv are suitably linked by a linker (Huston et al., Proc. Natl. Acad. Sci. USA, 85:5879-5883, (1988)) can be given as antibody fragments.

The antibodies may be conjugated to various molecules, such as fluorescent substances, radioactive substances, and luminescent substances. Methods to attach such moieties to an antibody are already established and conventional in the field (see, e.g., US 5,057,313 and 5,156,840).

Examples of methods that assay the antigen-binding activity of the antibodies include, for example, measurement of absorbance, enzyme-linked immunosorbent assay (ELISA), enzyme immunoassay (EIA), radioimmunoassay (RIA), and/or immunofluorescence. For example, when using ELISA, a protein encoded by a biomarker of the invention is added to a plate coated with the antibodies of the present disclosure, and then, the antibody sample, for example, culture supernatants of antibody-producing cells, or purified antibodies are added. Then, secondary antibody recognizing the primary antibody, which is labeled by alkaline phosphatase and such enzymes, is added, the plate is incubated and washed, and the absorbance is measured to evaluate the antigen-binding activity after adding an enzyme substrate such as p-nitrophenyl phosphate. As the protein, a protein fragment, for example, a fragment comprising a C-terminus, or a fragment comprising an N-terminus may be used. To evaluate the activity of the antibody of the invention, BIAcore (GE Healthcare) may be used.

By using these methods, the antibody of the invention and a sample presumed to contain a protein to be measured are contacted, and the protein is detected or assayed by detecting or assaying the immune complex formed between the above-mentioned antibody and the protein.

Mass spectrometry based quantitation assay methods, for example, but not limited to, multiple reaction monitoring (MRM)-based approaches in combination with stable-isotope labeled internal standards, are an alternative to immunoassays for quantitative measurement of proteins. These approaches do not require the use of antibodies and so the analysis can be performed in a cost- and time- efficient manner (see, for example, Addona et al., Nat. Biotechnol., 27:633-641, 2009; Kuzyk et al., Mol. Cell Proteomics, 8:1860-1877, 2009; Paulovich et al., Proteomics Clin. Appl., 2:1386-1402, 2008). In addition, MRM offers superior multiplexing capabilities, allowing for the simultaneous quantification of numerous proteins in parallel. The basic theory of these methods has been well-established and widely utilized for drug metabolism and pharmacokinetics analysis of small molecules.

A variety of suitable methods can be employed to detect and/or measure the level of mRNA expression of a gene. For example, mRNA expression can be determined using Northern blot or dot blot analysis, reverse transcriptase-PCR (RT-PCR; e.g., quantitative RT-PCR), in situ hybridization (e.g., quantitative in situ hybridization) or nucleic acid array (e.g., oligonucleotide arrays or gene chips) analysis. Details of such methods are described below and in, e.g., Sambrook et al., Molecular Cloning: A Laboratory Manual Second Edition vol. 1, 2 and 3. Cold Spring Harbor Laboratory Press: Cold Spring Harbor, New York, USA, Nov. 1989; Gibson et al. (1999) Genome Res., 6(10):995-100i; and Zhang et al. (2005) Environ. Sci. Technol., 39(8):2777-2785; U.S. Publication No. 2004086915; European Patent No. 0543942; and U.S. Patent No. 7,101,663.

In one embodiment, the presence or amount of discrete populations of mRNA populations in a biological sample can be determined by isolating total mRNA from the biological sample (see, e.g., Sambrook et al. (supra) and U.S. Patent No. 6,812,341) and subjecting the isolated mRNA to agarose gel electrophoresis to separate the mRNA by size. The size-separated mRNAs are then transferred (e.g., by diffusion) to a solid support such as a nitrocellulose membrane. The presence or amount of mRNA populations in the biological sample can then be determined using one or more detectably-labeled-polynucleotide probes, complementary to the mRNA sequence of interest, which bind to and thus render detectable their corresponding mRNA populations. Detectable-labels include, e.g., fluorescent (e.g., umbelliferone, fluorescein, fluorescein isothiocyanate, rhodamine, dichlorotriazinylamine fluorescein, dansyl chloride, allo-phycocyanin (APC), or phycoerythrin), luminescent (e.g., europium, terbium, Qdot^{™} nanoparticles supplied by the Quantum Dot Corporation, Palo Alto, CA), radiological (e.g., 1251, 1311, 35S, 32P, 33P, or 3H), and enzymatic (horseradish peroxidase, alkaline phosphatase, beta-galactosidase, or acetylcholinesterase) labels.

In another embodiment, the presence or amount of discrete populations of mRNA in a biological sample can be determined using nucleic acid (or oligonucleotide) arrays (e.g., an array described below under "Arrays and Kits"). For example, isolated mRNA from a biological sample can be amplified using RT-PCR with, e.g., random hexamer or oligo(dT)-primer mediated first strand synthesis. The amplicons can be fragmented into shorter segments. The RT-PCR step can be used to detectably-label the amplicons, or, optionally, the amplicons can be detectably-labeled subsequent to the RT-PCR step. For example, the detectable-label can be enzymatically (e.g., by nick-translation or kinase such as T4 polynucleotide kinase) or chemically conjugated to the amplicons using any of a variety of suitable techniques (see, e.g., Sambrook et al., supra). The detectably-labeled-amplicons are then contacted with a plurality of polynucleotide probe sets, each set containing one or more of a polynucleotide (e.g., an oligonucleotide) probe specific for (and capable of binding to) a corresponding amplicon, and where the plurality contains many probe sets each corresponding to a different amplicon.

Generally, the probe sets are bound to a solid support and the position of each probe set is predetermined on the solid support. The binding of a detectably-labeled amplicon to a corresponding probe of a probe set indicates the presence or amount of a target mRNA in the biological sample. Additional methods for detecting mRNA expression using nucleic acid arrays are described in, e.g., U.S. Patent Nos. 5,445,934; 6,027,880; 6,057,100; 6,156,501; 6,261,776; and 6,576,424.

Methods of detecting and/or for quantifying a detectable label depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

### Creating A Response Profile

The methods described herein can also be used to generate a response profile for a subject having renal cell carcinoma to a combination therapy comprising lenvatinib compound (e.g., lenvatinib mesylate) and everolimus. The profile can include, e.g., information that indicates the baseline expression level of the proteins required to be measured before the treatment with lenvatinib or a pharmaceutically acceptable salt thereof; and/or the histological analysis of any renal cell carcinoma. The resultant information (lenvatinib therapy response profile) can be used for predicting that a subject (e.g., a human patient) having, suspected of having or at risk of developing renal cell carcinoma is responsive to a combination therapy comprising a lenvatinib compound (e.g., lenvatinib mesylate) and everolimus.

It is understood that a lenvatinib compound (e.g., lenvatinib mesylate) response profile can be in electronic form (e.g., an electronic patient record stored on a computer or other electronic (computer-readable) media such as a DVD, CD, or floppy disk) or written form. The lenvatinib compound (e.g., lenvatinib mesylate) response profile can also include information for several (e.g., two, three, four, five, 10, 20, 30, 50, or 100 or more) subjects (e.g., human patients). Such multi-subject response profiles can be used, e.g., in analyses (e.g., statistical analyses) of particular characteristics of subject cohorts.

Responsiveness of a subject to a combination therapy comprising lenvatinib or a pharmaceutically acceptable salt thereof (e.g., lenvatinib mesylate) and everolimus can be classified in several ways and classification is dependent on the subject's disease, the severity of the disease, and the particular medicament the subject is administered. In the simplest sense, responsiveness is any decrease in the disease state as compared to pre-treatment, and non-responsiveness is the lack of any change in the disease state as compared to pre-treatment. Responsiveness of a subject (e.g., a human) with a renal cell carcinoma can be classified based on one or more of a number of objective clinical indicia such as, but not limited to, tumor size, Clinical Benefit (CB), PFS, OS, % of maximum tumor Shrinkage MTS, or ORR.

"Clinical benefit" refers to having one of the following statuses - Complete Response (CR), Partial Response (PR); or Stable Disease (SD) with 6 months or more PFS. "Complete Response" means complete disappearance of all target lesions. "Partial Response" means at least 30% decrease in the sum of the LD of target lesions, taking as reference the baseline summed LD. "Progressive Disease" (PD) means at least 20% increase in the sum of the LD of target lesions, taking as reference the smallest summed LD recorded since the treatment started, or the appearance of one or more new lesions. "Stable Disease" means neither sufficient shrinkage of the target lesions to qualify for PR nor sufficient increase to qualify for PD, taking as reference the smallest summed LD since the treatment started.

"Overall Survival" (OS) is defined as the time from randomization until death from any cause. "Randomization" means randomization of a patient into a test group or a control group when therapy plan for a patient is determined.

"Progression Free Survival" (PFS) refers to the time from the date of randomization to the date of first documentation of disease progression or death, whichever occurs first.

"% of Maximum Tumor shrinkage" (MTS) means percent change of sum of diameters of target lesions, taking as reference the baseline sum diameters.

"Objective Response Rate" (ORR) compares subjects with either CR or PR with subjects with either SD or PD.

"Better therapeutic outcome" can be evaluated based on tumor size, CB, PFS, OS, % of MTS, or ORR. In one embodiment, the better therapeutic outcome is longer PFS or longer OS.

### Kits

This application also provides kits. In certain embodiments, the kit can include an antibody or antibodies that can be used to detect the protein required to be measured or its concentration or expression levels. The antibodies in the kit may be monoclonal or polyclonal and can be further conjugated with a detectable label. The kits can, optionally, contain instructions for detecting and/or measuring the concentration of the protein required to be measured in a biological sample.

The kits can optionally include, e.g., a control (e.g., a concentration standard for the protein required to be measured). In some instances, the control can be an insert (e.g., a paper insert or electronic medium such as a CD, DVD, or floppy disk) containing an expression level or expression level ranges of the protein which is predictive of a necessity for a combination therapy comprising a lenvatinib compound (e.g., lenvatinib mesylate) and everolimus in the single biomarker analysis or which is correlated with a population of human subjects with better therapeutic outcome in the composite analysis. The kit for the composite analysis can optionally include an insert containing a control composite biomarker score value or range which is predictive of a necessity for a combination therapy comprising a lenvatinib compound (e.g., lenvatinib mesylate) and everolimus.

In some embodiments, the kits can include one or more reagents for processing a biological sample (e.g., calibration reagents, buffers, diluents, color reagents, reagents to stop a reaction). For example, a kit can include reagents for isolating a protein from a biological sample and/or reagents for detecting the presence and/or amount of the protein required to be measured in a biological sample (e.g., an antibody that binds to the protein and/or an antibody that binds the antibody that binds to the protein).

In certain embodiments, the kit includes at least one microplate (e.g., a 96 well plate; i.e., 12 strips of 8 wells). The microplate can be provided with its corresponding plate cover. The microplate can be polystyrene or of any other suitable material. The microplate can have the antibody that is used to identify the presence of a protein to be measured coated inside each well. The antibody may be conjugated to a detectable label. The kit may also include at least one adhesive strip.

In some embodiments, the kits can include a software package for analyzing the results of, e.g., expression profile or a microarray analysis.

The kits described herein can also, optionally, include instructions for administering a combination therapy comprising a lenvatinib compound and everolimus, where the baseline expression level of at least one protein selected from the group consisting of IL-18BP, ICAM-1, FGF-21 and M-CSF or the composite biomarker score calculated based on the baseline expression levels of:
(A) at least five proteins comprising (i) HGF, MIG, IL-18BP, IL-18 and ANG-2, or (ii) TIMP-1, M-CSF, IL-18BP, ANG-2 and VEGF-A, or
(B) at least two proteins comprising IL-18BP and ANG-2
   predicts that a subject having, suspected of having or at risk of developing renal cell carcinoma is responsive to a combination therapy comprising a lenvatinib compound (e.g., lenvatinib mesylate) and everolimus.

### Examples

### Example 1: Measuring biomarker expression levels in patients received a treatment with lenvatinib and/or everolimus

A Phase 2 study for lenvatinib was performed in patients with metastatic renal cell carcinoma (RCC) following a VEGF-targeted therapy. Patients having metastatic renal cell carcinoma after a prior VEGF-targeted therapy received lenvatinib mesylate ("lenvatinib"), everolimus, or the combination thereof until disease progression or development of unmanageable toxicities. A total of 153 subjects were randomized into 3 treatment arms to receive:
1) 18 mg/day lenvatinib (as lenvatinib free base; the same shall apply hereinafter) + 5 mg/day everolimus (Arm A [LEN/EVE treatment arm], N=51),
2) lenvatinib 24 mg/day (Arm B [LEN treatment arm], N=52), or
3) everolimus 10 mg/day (Arm C [EVE treatment arm], N=50).

### Each Cycle consisted of 4 weeks (28 days)

Blood samples for biomarker assessments were collected from subjects in all 3 treatment arms (LEN/EVE, N=49; LEN, N=51; EVE, N=47; Total, N=147) at Cycle 1 Day 1(pre-treatment). In samples from 145 subjects (94.8% of all Intention to Treat [ITT] population; LEN/EVE, N=49; LEN, N=50; EVE, N=46), 40 candidate biomarkers shown in Table 1 were assayed using 19 preconfigured CustomMAP immunoassay panels and measured by a multiplex flow cytometry-based platform by the manufacturer (Multi-Analyte Profile (MAP)) at a Myriad RBM (Austin, TX, USA). The results were further analyzed in correlation analyses described in examples below.

### [Table 1]

**Table 1. 40 Candidate Biomarkers**

| Abbreviation | Name of biomarker |
|---|---|
| ANG-1 | Angiopoietin 1 |
| ANG-2 | Angiopoietin 2 |
| CRP | C-reactive protein |
| EOTAXIN1 | eotaxin-1 |
| FGF-21 | Fibroblast growth factor 21 |
| FGF-23 | Fibroblast growth factor 23 |
| G-CSF | granulocyte colony-stimulating factor |
| HGF | hepatocyte growth factor |
| ICAM-1 | intercellular adhesion molecule-1 |
| IFN-gamma | Interferon gamma |
| IL-10 | Interleukin 10 |
| IL-12P70 | interleukin 12 subunit p70 |
| IL-13 | Interleukin 13 |
| IL-18 | Interleukin 18 |
| IL-18BP | Interleukin 18 binding protein |
| IL-1beta | Interleukin 1 beta |
| IL-4 | Interleukin 4 |
| IL-6 | Interleukin 6 |
| IL-8 | Interleukin 8 |
| IP-10 | Interferon gamma induced protein 10 |
| ITAC | interferon-inducible T-cell alpha chemoattractant |
| KIM-1 | kidney injury molecule 1 |
| M-CSF | macrophage colony-stimulating factor |
| MCP-1 | monocyte chemotactic protein 1 |
| MIG | monokine induced by gamma interferon |
| MIP-1 alpha | macrophage inflammatory protein 1 alpha |
| MMP-9 | matrix metalloproteinase 9 |
| PLGF | placenta growth factor |
| RANTES | regulated on activation, normal T cell expressed and secreted |
| SDF-1 | stromal cell-derived factor 1 |
| TIE-2 | tyrosine kinase with immunoglobulin and epidermal growth factor homology domains 2 |
| TIMP-1 | tissue inhibitor of metalloproteinases 1 |
| TNF alpha | tumor necrosis factor alpha |
| TNFR2 | Tumor necrosis factor receptor 2 |
| VCAM-1 | vascular cell adhesion molecule 1 |
| VEGF-A | vascular endothelial growth factor A |
| VEGF-D | vascular endothelial growth factor D |
| VEGFR-1 | vascular endothelial growth factor receptor 1 |
| VEGFR-2 | vascular endothelial growth factor receptor 2 |
| VEGFR-3 | vascular endothelial growth factor receptor 3 |

### Example 2: Single Biomarker Analysis With PFS and OS

For baseline levels of each serum biomarker, correlation analyses with PFS were performed using univariate Cox regression in each of the 3 arms. Hazard ratio (HR) for biomarkers with continuous values was calculated as a ratio of hazards between measured values with one standard deviation (HR per standard deviation). Baseline serum IL-18BP levels were associated with PFS in the LEN/EVE arm (Table 2). In the following tables, FDR refers to False Discovery Rate, MST refers to Median Survival Time and CI refers to Confidence Interval.

### [Table 2]

**Table 2: Univariate Cox Regression Analysis of Baseline Levels of IL-18BP With PFS in LEN/EVE and EVE Arms**

| Marker | LEN/EVE | | | | EVE | | | |
|---|---|---|---|---|---|---|---|---|
| | N | HR (95% Cl) | P value | P value with FDR | N | HR (95% Cl) | P value | P value with FDR |
| IL-18BP | 48 | 1.720 (1.226, 2.413) | 0.0017 | 0.0457 | 46 | 1.207 (0.837, 1.740) | 0.3149 | 0.8858 |

For IL-18BP, dichotomized analysis was performed to correlate baseline serum biomarker levels in subjects with low and high levels with PFS in the 3 arms. The cutoff points were median, first tertile, second tertile, first quartile or third quartile. Association between the groups and PFS was explored using univariate Cox regression and the log-rank test with each of the selected cutoff points. At the second tertile cutoff point, median PFS for the high IL-18BP group (5.6 months) was shorter than that for the low group (17.5 months) in the LEN/EVE arm (Table 3). Multivariate Cox regression analysis with treatment arm, baseline level and PFS showed interactions at the second tertile cutoff point compared to the EVE arm (interaction P value=0.0389). These results suggested that low IL-18BP levels had potential predictive roles for longer PFS in the LEN/EVE arm compared to the EVE arm.

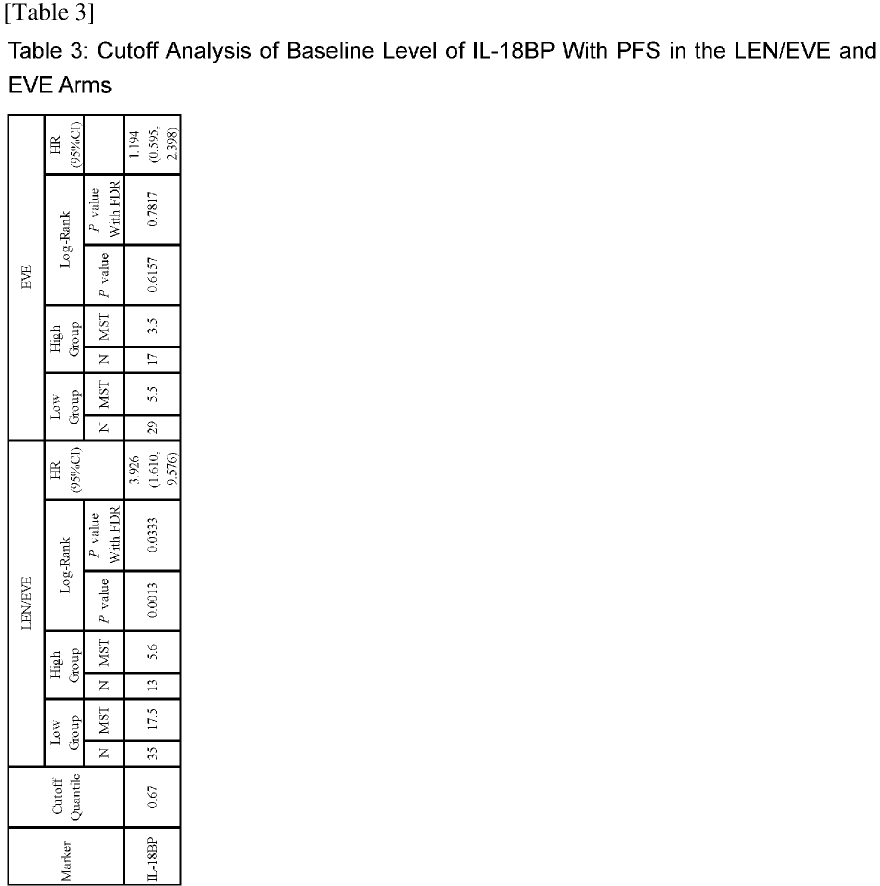

For baseline levels of each serum biomarker, correlation analyses with OS were performed using univariate Cox regression in each of the 3 arms. Hazard ratio (HR) for biomarkers with continuous values was calculated as a ratio of hazards between measured values with one standard deviation (HR per standard deviation). Baseline serum FGF-21, ICAM-1, and M-CSF levels were associated with OS in the LEN/EVE arm (Table 4).

### [Table 4]

**Table 4: Univariate Cox Regression Analysis of Baseline Levels of FGF-21, ICAM-1, and M-CSF With OS in LEN/EVE and EVE Arms**

| Marker | LEN/EVE | | | | EVE | | | |
|---|---|---|---|---|---|---|---|---|
| | N | HR (95% CI) | P value | P value with FDR | N | HR (95% CI) | P value | P value with FDR |
| FGF-21 | 48 | 1.255 (1.053, 1.496) | 0.0114 | 0.0307 | 46 | 0.996 (0.615, 1.612) | 0.9860 | 0.9860 |
| ICAM-1 | 48 | 1.505 (1.116, 2.028) | 0.0073 | 0.0219 | 46 | 1.017 (0.688, 1.502) | 0.9339 | 0.9698 |
| M-CSF | 48 | 1.784 (1.254, 2.539) | 0.0013 | 0.0058 | 46 | 1.366 (0.995, 1.875) | 0.0539 | 0.1456 |

For FGF-21, ICAM-1, and M-CSF, dichotomized analyses were performed to correlate baseline serum biomarker levels in subjects with low and high levels with OS in the 3 arms using univariate Cox regression and the log-rank test at median cutoff point. Low baseline levels of FGF-21, ICAM-1, and M-CSF were associated with longer OS in the LEN/EVE arm (Table 5). Multivariate Cox regression analysis with treatment arm, baseline level and OS showed interactions at the median cutoff point compared to the EVE arm (interaction P value= 0.0223, 0.0039, and 0.0362 for FGF-21, ICAM-1, and M-CSF, respectively). These results suggested that low FGF-21, ICAM-1, and M-CSF levels had potential predictive roles for longer OS in the LEN/ EVE arm compared to the EVE arm.

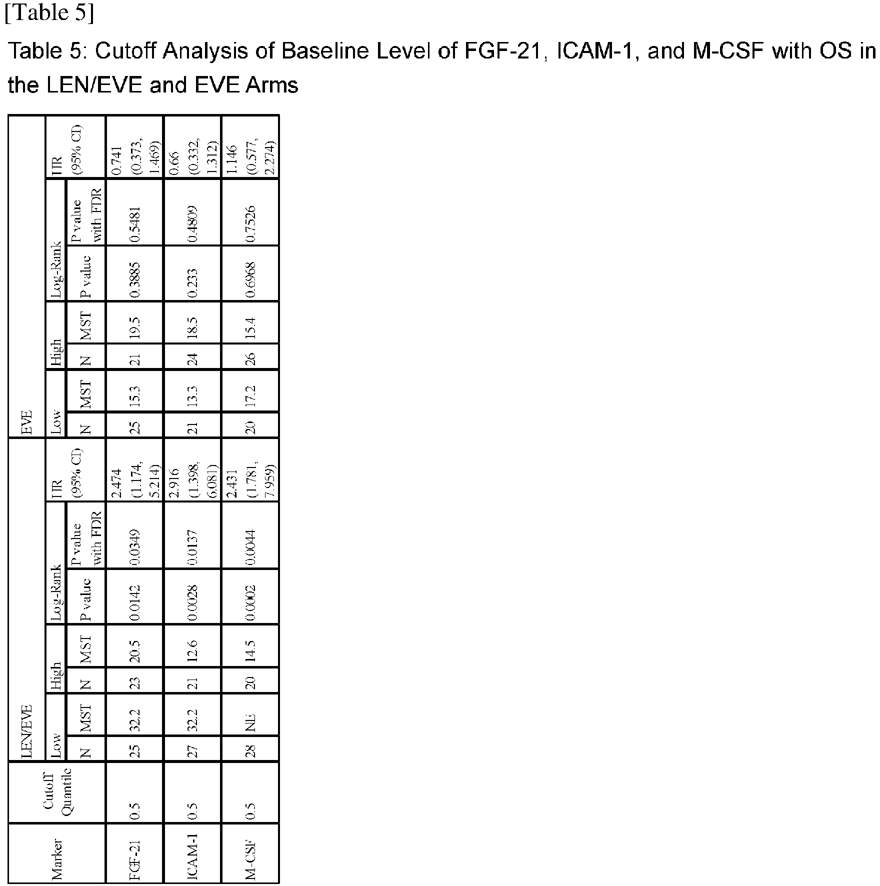

### Example 3: Composite Biomarker Score Analysis (5 markers) With PFS

According to Voss, et al., 2016 (Br J Cancer. 2016 Mar 15;114(6):642-9), composite biomarker score (CBS) analyses were conducted using 5 selected biomarkers (HGF, MIG, IL-18BP, IL-18, and ANG-2) of which baseline levels showed the strongest association in the cutoff analysis by median with PFS in the LEN/EVE arm (Table 6).

### [Table 6]

**Table 6. Associations of baseline biomarker levels with PFS in LEN+EVE arm**

| Marker | Cutoff | | Low Group | | High Group | | Log rank | | HR (95% CI) |
|---|---|---|---|---|---|---|---|---|---|
| | Quantile | Value | N | Median PFS (Months) | N | Median PFS (Months) | *P* value | With FDR | |
| HGF | 0.50 | 7.350 µg/L | 28 | 20.1 | 20 | 5.6 | 0.0264 | 0.1508 | 2.550 (1.088, 5.974) |
| MIG | 0.50 | 1250 ng/L | 30 | 20.1 | 18 | 7.4 | 0.0299 | 0.1508 | 2.506 (1.062, 5.914) |
| IL-18BP | 0.50 | 17.0 µg/L | 24 | 17.5 | 24 | 6.9 | 0.0305 | 0.1508 | 2.431 (1.059, 5.580) |
| IL-18 | 0.50 | 264.0 ng/L | 28 | 14.7 | 20 | 5.6 | 0.0317 | 0.1508 | 2.418 (1.058, 5.526) |
| ANG-2 | 0.50 | 6.800 µg/L | 27 | 20.1 | 21 | 5.9 | 0.0351 | 0.1508 | 2.360 (1.040, 5.355) |

For each biomarker integrated into the CBS, a value of 1 was assigned if the respective baseline biomarker level fell within the range determined to associate with longer survival in the single biomarker cutoff analysis; a value of 0 was assigned if the respective baseline biomarker level was categorized in the range associated with shorter survival. The sum of the individual values (0 vs. 1 for each of the 5 biomarkers) was computed as a CBS for each subject (range 0-5). Patients were then dichotomized per CBS as low and high groups (0 vs. 1-5, 0-1 vs. 2-5, 0-2 vs. 3-5, 0-3 vs. 4-5, and 0-4 vs. 5). Associations between the groups and PFS were explored using univariate Cox regression and the log-rank test with each of the selected dichotomization points in each treatment arm. Interaction P values were estimated by multivariate Cox regression analysis with treatment arm, baseline level and PFS. Associations between the groups and objective response rate (ORR) were also explored using Fisher's exact test.

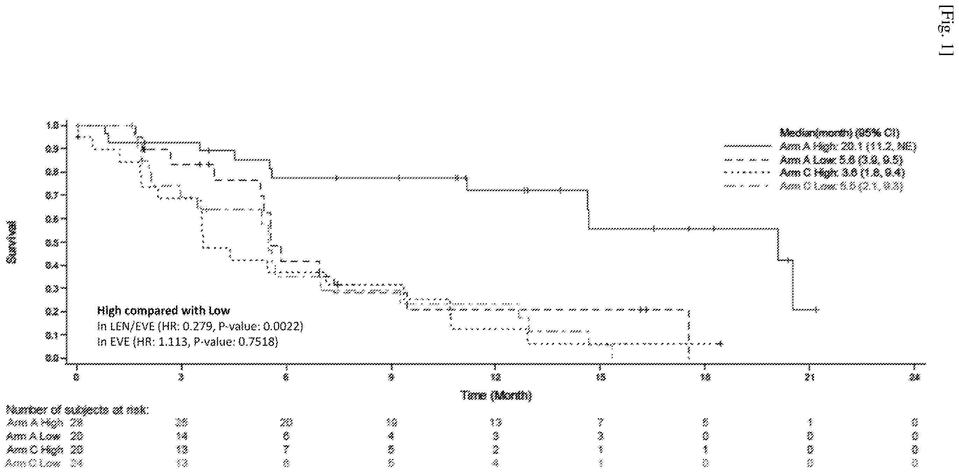

There was a significant difference in PFS between the CBS low and high groups in the LEN/EVE arm while there were no significant differences in PFS between the CBS low and high groups in the EVE arm (Figure 1). In the LEN/EVE arm, PFS was longer in patients with a high CBS score (3-5) than in patients with a low CBS scores (0-2) (mPFS: 20.1 months for high CBS and 5.6 months for low CBS, HR: 0.279, P value: 0.0022). In the patients with a high CBS score (3-5), PFS was longer in the LEN/EVE arm than in the EVE arm (mPFS: 20.1 months in LEN/EVE and 3.6 months in EVE, HR: 0.186, P value: <0.0001, Table 7).

Multivariate Cox regression analysis showed a significant interaction between PFS with LEN+EVE vs EVE and CBS group (P = 0.0154).

In the patients with a high CBS score (3-5), ORR was higher in the LEN/EVE (57.1%) than in the EVE arms (5.0%) (P value=0.0002, Table 7).

### [Table 7]

**Table 7: Median PFS and ORR in LEN+EVE and EVE arms by low (0-2) and high (3-5) CBS category**

| | CBS Low (0-2) | | | CBS High (3-5) | | |
|---|---|---|---|---|---|---|
| | LEN/EVE | EVE | *P*-value | LEN/EVE | EVE | *P*-value |
| | (N=20) | (N=24) | | (N=28) | (N=20) | |
| mPFS | 5.6 mo | 5.5 mo | 0.2387 | 20.1 mo | 3.6 mo | < 0.0001 |
| ORR | 30.0 % | 4.2 % | 0.0353 | 57.1 % | 5.0% | 0.0002 |

These data show high CBS was correlated with PFS benefit, and the score can be used to identify patients who clearly benefit from combination therapy compared with EVE monotherapy.

### Example 4: Composite Biomarker Score Analysis (5 markers) With OS

Similar to Example 3, CBS analyses were conducted using 5 selected biomarkers (TIMP-1, M-CSF, IL-18BP, ANG-2, and VEGF-A) of which baseline levels showed the strongest association in the cutoff analysis by median with OS in the LEN/EVE arm (Table 8).

### [Table 8]

**Table 8. Associations of baseline CAF levels with OS in LEN+EVE arm**

| Marker | Cutoff | | Low Group | | High Group | | Log rank | | HR (95% CI) |
|---|---|---|---|---|---|---|---|---|---|
| | Quantile | Value | N | Median OS (Months) | N | Median OS (Months) | *P* value | With FDR | |
| TIMP-1 | 0.500 | 199.0 µg/L | 26 | NE | 22 | 16.1 | 0.0003 | 0.0044 | 3.770 (1.741, 8.162) |
| M-CSF | 0.500 | 0.9650 µg/L | 28 | NE | 20 | 14.5 | 0.0002 | 0.0044 | 3.765 (1.781, 7.959) |
| IL-18BP | 0.500 | 17.00 µg/L | 24 | 32.2 | 24 | 18.4 | 0.0008 | 0.0073 | 3.469 (1.605, 7.501) |
| ANG-2 | 0.500 | 6.800 µg/L | 27 | NE | 21 | 21.7 | 0.0030 | 0.0137 | 3.005 (1.402, 6.442) |
| VEGF-A | 0.500 | 305.0 ng/L | 29 | 32.2 | 19 | 20.5 | 0.0021 | 0.0137 | 2.993 (1.441, 6.213) |

For each biomarker integrated into the CBS, a value of 1 was assigned if the respective baseline biomarker level fell within the range previously determined to associate with longer survival on single biomarker cutoff analysis; a value of 0 was assigned if the respective baseline biomarker level was categorized in the range associated with shorter survival. The sum of the individual values (0 vs. 1 for each of the 5 biomarkers) was computed as a CBS for each subject (range 0-5; high = favorable survival). Patients were then dichotomized per CBS as low and high groups (0 vs. 1-5, 0-1 vs. 2-5, 0-2 vs. 3-5, 0-3 vs. 4-5, and 0-4 vs. 5). Associations between the groups and OS were explored using univariate Cox regression and the log-rank test with each of the selected dichotomization points in each treatment arm. Interaction P values were estimated by multivariate Cox regression analysis with treatment arm, baseline level and OS. Associations between the groups and objective response rate (ORR) were also explored using Fisher's exact test.

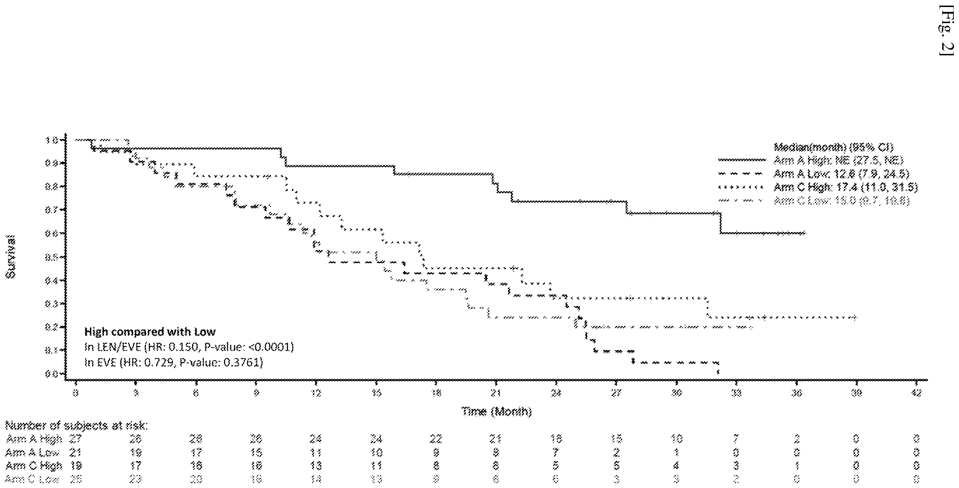

There was a significant difference in OS between the CBS low and high groups in the LEN/EVE arm while there were no significant differences in OS between the CBS low and high groups in the EVE arm (Figure 2). In the LEN/EVE arm, OS was longer in patients with a high CBS score (3-5) than in patients with a low CBS scores (0-2) (median OS (mOS): not evaluable (NE) for high CBS and 12.6 months for low CBS, HR=0.150, P value<0.0001). In the patients with a high CBS score (3-5), OS was longer in the LEN/EVE arm than in the EVE arm (mOS: NE in LEN/EVE and 17.4 months in EVE, HR: 0.331, P value: 0.0079, Table 9).

Multivariate Cox regression analysis showed a significant interaction between PFS with LEN+EVE vs EVE and CBS group (P = 0.0125).

In the patients with a high CBS score (3-5), ORR was higher in the LEN/EVE (63.0%) than in the EVE arms (5.3%) (P value<0.0001, Table 9).

### [Table 9]

**Table 9: Median OS and ORR in LEN+EVE and EVE arms by low (0-2) and high (3-5) CBS category**

| | CBS Low (0-2) | | | CBS High (3-5) | | |
|---|---|---|---|---|---|---|
| | LEN/EVE | EVE | *P*-value | LEN/EVE | EVE | *P*-value |
| | (N=21) | (N=25) | | (N=27) | (N=19) | |
| mOS | 12.6 mo | 15.0 mo | 0.5560 | NE | 17.4 mo | 0.0079 |
| ORR | 23.8% | 4.0% | 0.0790 | 63.0 % | 5.3 % | < 0.0001 |

These data show that high CBS was correlated with OS benefit, and the score can be used to identify patients who clearly benefit from combination therapy compared with EVE monotherapy.

### Example 5: Composite Biomarker Score Analysis (2 markers) With PFS

IL-18BP and ANG-2 were common in biomarkers selected for CBS analysis for PFS and OS in Example 3 and 4. Using these 2 biomarkers, CBS analysis (2 markers) with PFS was performed.

For each biomarker integrated into the CBS, a value of 1 was assigned if the respective baseline biomarker level fell within the range determined to associate with longer survival in the single biomarker cutoff analysis; a value of 0 was assigned if the respective baseline biomarker level was categorized in the range associated with shorter survival. The sum of the individual values (0 vs. 1 for each of the 2 biomarkers) was computed as a CBS for each subject (range 0-2). Patients were then dichotomized per CBS as low and high groups (0 vs. 1-2 and 0-1 vs. 2). Associations between the groups and PFS were explored using univariate Cox regression and the log-rank test with each of the selected dichotomization points in each treatment arm. Interaction P values were estimated by multivariate Cox regression analysis with treatment arm, baseline level and PFS. Associations between the groups and objective response rate (ORR) were also explored using Fisher's exact test.

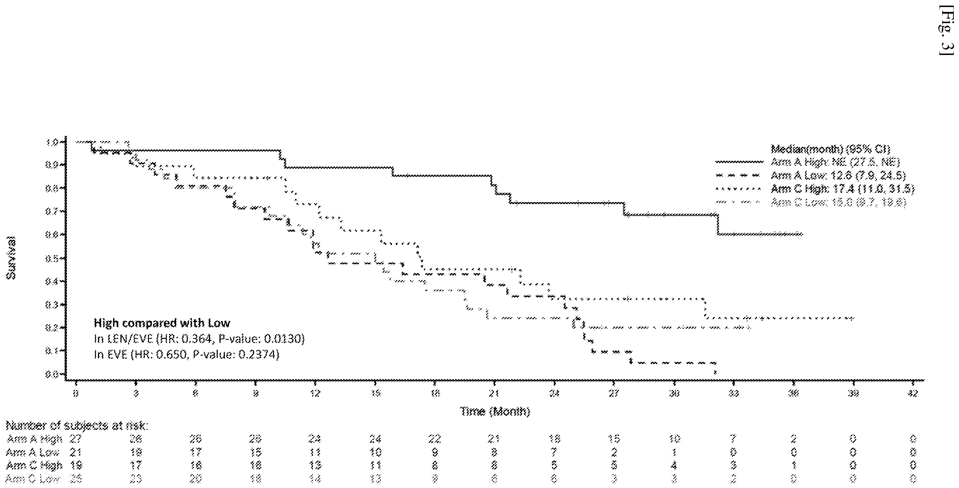

There was a significant difference in PFS between the CBS low and high groups in the LEN/EVE arm while there were no significant differences in PFS between the CBS low and high groups in the EVE arm (Figure 3). In the LEN/EVE arm, PFS was longer in patients with a high CBS score (1-2) than in patients with a low CBS scores (0) (mPFS: 17.5 months for high CBS and 5.6 months for low CBS, HR=0.364, P value=0.0130). In the patients with a high CBS score (1-2), PFS was longer in the LEN/EVE arm than in the EVE arm (mPFS: 17.5 months in LEN/EVE and 5.5 months in EVE, HR: 0.254, P value: <0.0001, Table 10).

Multivariate Cox regression analysis showed no significant interaction between PFS with LEN+EVE vs EVE and CBS group (P = 0.2297).

In the patients with a high CBS score (1-2), ORR was higher in the LEN/EVE (54.3%) than in the EVE arms (6.9%) (P value<0.0001, Table 10).

### [Table 10]

**Table 10: Median PFS and ORR in LEN+EVE and EVE arms by low (0) and high (1-2) CBS category**

| | CBS Low (0) | | | CBS High (1-2) | | |
|---|---|---|---|---|---|---|
| | LEN/EVE | EVE | *P*-value | LEN/EVE | EVE | *P*-value |
| | (N=13) | (N=15) | | (N=35) | (N=29) | |
| mPFS | 5.6 mo | 3.0 mo | 0.2178 | 17.5 mo | 5.5 mo | < 0.0001 |
| ORR | 23.1 % | 0.0 % | 0.0873 | 54.3% | 6.9 % | < 0.0001 |

### Example 6: Composite Biomarker Score Analysis (2 markers) With OS

IL-18BP and ANG-2 were common in biomarkers selected for CBS analysis for PFS and OS in Example 3 and 4. Using these 2 biomarkers, CBS analysis (2 markers) with OS was performed.

For each biomarker integrated into the CBS, a value of 1 was assigned if the respective baseline biomarker level fell within the range determined to associate with longer survival in the single biomarker cutoff analysis; a value of 0 was assigned if the respective baseline biomarker level was categorized in the range associated with shorter survival. The sum of the individual values (0 vs. 1 for each of the 2 biomarkers) was computed as a CBS for each subject (range 0-2). Patients were then dichotomized per CBS as low and high groups (0 vs. 1-2 and 0-1 vs. 2). Associations between the groups and OS were explored using univariate Cox regression and the log-rank test with each of the selected dichotomization points in each treatment arm. Interaction P values were estimated by multivariate Cox regression analysis with treatment arm, baseline level and OS.

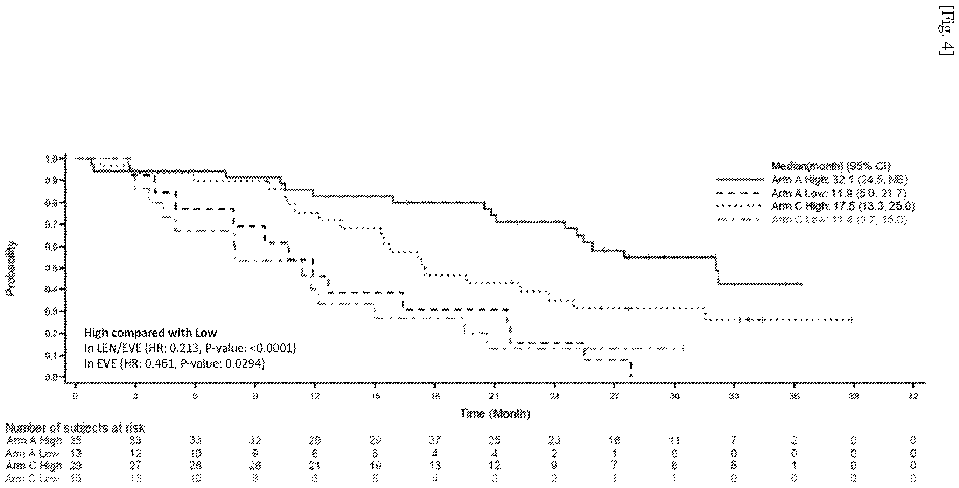

There was a significant difference in OS between the CBS low and high groups in the LEN/EVE and the EVE arms (Figure 4). In the LEN/EVE arm, OS was longer in patients with a high CBS score (1-2) than in patients with a low CBS scores (0) (mOS: 32.1 months for high CBS and 11.9 months for low CBS, HR=0.213, P value<0.0001). In the EVE arm, OS was longer in patients with a high CBS score (1-2) than in patients with a low CBS scores (0) (mOS: 17.5 for high CBS and 11.4 months for low CBS, HR=0.461, P value=0.0294). In the patients with a high CBS score (1-2), OS was longer in the LEN/EVE arm than in the EVE arm (mOS: 32.1 months in LEN/EVE and 17.5 months in EVE, HR: 0.504, P value: 0.0359, Table 11).

Multivariate Cox regression analysis showed no significant interaction between OS with LEN+EVE vs EVE and CBS group (P = 0.2125).

### [Table 11]

**Table 11: Median OS in LEN+EVE and EVE arms by low (0) and high (1-2) CBS category**

| | CBS Low (0) | | | CBS High (1-2) | | |
|---|---|---|---|---|---|---|
| | LEN/EVE | EVE | *P*-value | LEN/EVE | EVE | *P*-value |
| | (N=13) | (N=15) | | (N=35) | (N=29) | |
| mOS | 11.9 mo | 11.4 mo | 0.8945 | 32.1 mo | 17.5 mo | 0.0359 |

## Claims

1. A method of predicting the response of a human subject having, suspected of having, or at risk of developing a renal cell carcinoma to a combination therapy comprising lenvatinib or a pharmaceutically acceptable salt thereof, preferably lenvatinib mesylate and everolimus, the method comprising measuring or having measured a baseline expression level of at least one protein selected from the group consisting of IL-18BP, ICAM-1, FGF-21 and M-CSF in a biological sample obtained from the human subject,
wherein preferably the baseline expression level of the at least one protein is determined by measuring the amount of the at least one protein in the biological sample or the baseline expression level of the at least one protein is determined by measuring the amount of mRNA encoding the at least one protein in the biological sample,
wherein a lower baseline expression level of the protein, as compared to a control expression level, of the at least one protein is predictive that the human subject is responsive to the combination therapy comprising lenvatinib or a pharmaceutically acceptable salt thereof, preferably lenvatinib mesylate and everolimus,
wherein the control expression level is an expression level in a biological sample obtained from a subject who is not responsive to the combination therapy comprising lenvatinib or a pharmaceutically acceptable salt thereof, preferably lenvatinib mesylate and everolimus, and
wherein the biological sample is a blood sample, preferably a serum sample, or a plasma sample.

2. The method of claim 1, wherein the renal cell carcinoma is an advanced renal cell carcinoma or a metastatic renal cell carcinoma.

3. A method of predicting the response of a human subject having, suspected of having, or at risk of developing a renal cell carcinoma to a combination therapy comprising lenvatinib or a pharmaceutically acceptable salt thereof, preferably lenvatinib mesylate and everolimus, the method comprising the steps of:
measuring or having measured baseline expression levels of at least five proteins in a biological sample obtained from the human subject,
wherein preferably the baseline expression level of the at least five proteins is determined by measuring the amount of each the at least five proteins in the biological sample or the baseline expression level of the at least five proteins is determined by measuring the amount of mRNAs encoding each of the at least five proteins in the biological sample;
determining a score for each protein based on its baseline expression level;
summing up the scores to obtain a composite biomarker score; and
determining that the human subject is responsive to the combination therapy based on the composite biomarker score;
wherein the at least five proteins comprise
(i) HGF, MIG, IL-18BP, IL-18 and ANG-2; or
(ii) TIMP-1, M-CSF, IL-18BP, ANG-2 and VEGF; and
wherein
(a) the score for each protein is larger if the baseline expression level falls within a value range that is correlated with a population of human subjects with better therapeutic outcome in response to the combination therapy, and the composite biomarker score equal to or higher than a control composite biomarker score is predictive that the human subject is responsive to the combination therapy comprising lenvatinib or a pharmaceutically acceptable salt thereof, preferably lenvatinib mesylate and everolimus; or
(b) the score for each protein is smaller if the baseline expression level falls within a value range that is correlated with a population of human subjects with better therapeutic outcome in response to the combination therapy, and the composite biomarker score equal to or lower than a control composite biomarker score is predictive that the human subject is responsive to the combination therapy comprising lenvatinib or a pharmaceutically acceptable salt thereof, preferably lenvatinib mesylate and everolimus,
wherein the control composite biomarker score is a composite biomarker score from a biological sample obtained from a subject who is not responsive to the combination therapy comprising lenvatinib or a pharmaceutically acceptable salt thereof, preferably lenvatinib mesylate and everolimus, and
wherein the biological sample is a blood sample, preferably a serum sample, or a plasma sample.

4. The method of claim 3, wherein the control composite biomarker score is a predetermined cut-off value.

5. The method of claim 3 or 4, wherein the score for each protein is a binary value based on the baseline expression level for each of the proteins.

6. The method of claim 5, wherein the binary value is 0 or 1.

7. The method of any one of claims 3 to 6, wherein the better therapeutic outcome is longer overall survival or longer progression free survival.

8. The method of any one of the claims 3 to 7, wherein the renal cell carcinoma is an advanced renal cell carcinoma or a metastatic renal cell carcinoma.

9. The method of any one of the claims 3 to 8, wherein the at least five proteins consists of HGF, MIG, IL-18BP, IL-18 and ANG-2.

10. The method of any one of the claims 3 to 8, wherein the at least five proteins consists of TIMP-1, M-CSF, IL-18BP, ANG-2 and VEGF-A.

11. A method of predicting the response of a human subject having, suspected of having, or at risk of developing a renal cell carcinoma to a combination therapy comprising lenvatinib or a pharmaceutically acceptable salt thereof, preferably lenvatinib mesylate and everolimus, the method comprising the steps of:
measuring or having measured baseline expression levels of at least two proteins comprising IL-18BP and ANG-2 in a biological sample obtained from the human subject,
wherein preferably the baseline expression level of each of the proteins is determined by measuring the amount of each of the proteins in the biological sample or the baseline expression level of each of the proteins is determined by measuring the amount of mRNAs encoding each of the proteins in the biological sample;
determining or having determined a score for each protein based on its baseline expression level;
summing up or having summed up the scores to obtain a composite biomarker score; and
wherein
(a) the score for each protein is larger if the baseline expression level falls within a value range that is correlated with a population of human subjects with better therapeutic outcome in response to the combination therapy, and the composite biomarker score equal to or higher than a control composite biomarker score is predictive that the human subject is responsive to the combination therapy comprising lenvatinib or a pharmaceutically acceptable salt thereof, preferably lenvatinib mesylate and everolimus; or
(b) the score for each protein is smaller if the baseline expression level falls within a value range that is correlated with a population of human subjects with better therapeutic outcome in response to the combination therapy, and the composite biomarker score equal to or lower than a control composite biomarker score is predictive that the human subject is responsive to the combination therapy comprising lenvatinib or a pharmaceutically acceptable salt thereof, preferably lenvatinib mesylate and everolimus,
wherein the control composite biomarker score is a composite biomarker score from a biological sample obtained from a subject who is not responsive to the combination therapy comprising lenvatinib or a pharmaceutically acceptable salt thereof, preferably lenvatinib mesylate and everolimus, and
wherein the biological sample is a blood sample, preferably a serum sample, or a plasma sample.

12. The method of claim 11, wherein the control composite biomarker score is a pre-determined cut-off value.

13. The method of claim 11 or 12, wherein the score for each protein is a binary value based on the baseline expression level for each of the proteins.

14. The method of claim 13, wherein the binary value is 0 or 1.

15. The method of claim 13 or 14, wherein the better therapeutic outcome is longer overall survival or longer progression free survival.

16. The method of any one of the claims 11 to 15, wherein the renal cell carcinoma is an advanced renal cell carcinoma or a metastatic renal cell carcinoma.

17. The method of any one of the claims 11 to 16, wherein the at least two proteins consists of IL-18BP and ANG-2.

## Patentansprüche

1. Verfahren zur Vorhersage der Reaktion eines menschlichen Subjekts, das ein Nierenzellkarzinom hat, von dem vermutet wird ein Nierenzellkarzinom zu haben, oder das gefährdet ist ein Nierenzellkarzinom zu entwickeln, auf eine Kombinationstherapie, die Lenvatinib oder ein pharmazeutisch akzeptables Salz davon umfasst, vorzugsweise Lenvatinibmesylat und Everolimus, das Verfahren umfassend Messen oder gemessen haben eines Basislinien-Expressionslevels mindestens eines Proteins, das aus der Gruppe bestehend aus IL-18BP, ICAM-1, FGF-21 und M-CSF ausgewählt ist, in einer biologischen Probe erhalten von dem menschlichen Subjekt,
wobei vorzugsweise das Basislinien-Expressionslevel des mindestens einen Proteins durch Messen der Menge des mindestens einen Proteins in der biologischen Probe bestimmt wird oder das Basislinien-Expressionslevel des mindestens einen Proteins durch Messen der Menge von mRNA, die für das mindestens eine Protein kodiert, in der biologischen Probe bestimmt wird,
wobei ein niedrigeres Basislinien-Expressionslevel des Proteins, im Vergleich zu einem Kontroll- Expressionslevel, des mindestens einen Proteins prädiktiv dafür ist, dass das menschliche Subjekt auf die Kombinationstherapie anspricht, die Lenvatinib oder ein pharmazeutisch akzeptables Salz davon umfasst, vorzugsweise Lenvatinibmesylat und Everolimus,
wobei das Kontroll-Expressionslevel ein Expressionslevel in einer biologischen Probe erhalten von einem Subjekt ist, das nicht auf die Kombinationstherapie anspricht, die Lenvatinib oder ein pharmazeutisch akzeptables Salz davon umfasst, vorzugsweise Lenvatinibmesylat und Everolimus, und
wobei die biologische Probe eine Blutprobe ist, vorzugsweise eine Serumprobe, oder eine Plasmaprobe.

2. Verfahren nach Anspruch 1, wobei das Nierenzellkarzinom ein fortgeschrittenes Nierenzellkarzinom oder ein metastasiertes Nierenzellkarzinom ist.

3. Verfahren zur Vorhersage der Reaktion eines menschlichen Subjekts, das ein Nierenzellkarzinom hat, von dem vermutet wird ein Nierenzellkarzinom zu haben, oder das gefährdet ist ein Nierenzellkarzinom zu entwickeln, auf eine Kombinationstherapie, die Lenvatinib oder ein pharmazeutisch akzeptables Salz davon umfasst, vorzugsweise Lenvatinibmesylat und Everolimus, das Verfahren umfassend die Schritte:
Messen oder gemessen haben der Basislinien-Expressionslevel von mindestens fünf Proteinen in einer biologischen Probe erhalten von dem menschlichen Subjekt,
wobei vorzugsweise das Basislinien-Expressionslevel der mindestens fünf Proteine durch Messen der Menge jedes der mindestens fünf Proteine in der biologischen Probe bestimmt wird oder das Basislinien-Expressionslevel der mindestens fünf Proteine durch Messen der Menge von mRNAs, die für jedes der mindestens fünf Proteine kodieren, in der biologischen Probe bestimmt wird;
Bestimmen einer Punktzahl (score) für jedes Protein aufgrund seines Basislinien-Expressionslevels;
Aufsummieren der Punktzahlen (scores), um eine zusammengesetzte Biomarker-Punktzahl (composite biomarker score) zu erhalten; und
Bestimmen, dass das menschliche Subjekt auf die Kombinationstherapie anspricht, aufgrund der zusammengesetzten Biomarker-Punktzahl (composite biomarker score);
wobei die mindestens fünf Proteine umfassen
(i) HGF, MIG, IL-18BP, IL-18 und ANG-2; oder
(ii) TIMP-1, M-CSF, IL-18BP, ANG-2 und VEGF; und
wobei
(a) die Punktzahl (score) für jedes Protein größer ist, wenn das Basislinien-Expressionslevel in einen Wertebereich fällt, der mit einer Population menschlicher Subjekte mit besserem therapeutischen Ergebnis als Reaktion auf die Kombinationstherapie korreliert ist, und die zusammengesetzte Biomarker-Punktzahl (composite biomarker score) gleich oder höher als eine zusammengesetzte Biomarker-Punktzahl (composite biomarker score) Kontrolle prädiktiv dafür ist, dass das menschliche Subjekt auf die Kombinationstherapie anspricht, die Lenvatinib oder ein pharmazeutisch akzeptables Salz davon umfasst, vorzugsweise Lenvatinibmesylat und Everolimus; oder
(b) die Punktzahl (score) für jedes Protein kleiner ist, wenn das Basislinien-Expressionslevel in einen Wertebereich fällt, der mit einer Population menschlicher Subjekte mit besserem therapeutischen Ergebnis als Reaktion auf die Kombinationstherapie korreliert ist, und die zusammengesetzte Biomarker-Punktzahl (composite biomarker score) gleich oder niedriger als eine zusammengesetzte Biomarker-Punktzahl (composite biomarker score) Kontrolle prädiktiv dafür ist, dass das menschliche Subjekt auf die Kombinationstherapie anspricht, die Lenvatinib oder ein pharmazeutisch akzeptables Salz davon umfasst, vorzugsweise Lenvatinibmesylat und Everolimus,
wobei die zusammengesetzte Biomarker-Punktzahl (composite biomarker score) Kontrolle eine zusammengesetzte Biomarker-Punktzahl (composite biomarker score) aus einer biologischen Probe erhalten von einem Subjekt ist, das nicht auf die Kombinationstherapie anspricht, die Lenvatinib oder ein pharmazeutisch akzeptables Salz davon umfasst, vorzugsweise Lenvatinibmesylat und Everolimus, und
wobei die biologische Probe eine Blutprobe ist, vorzugsweise eine Serumprobe, oder eine Plasmaprobe.

4. Verfahren nach Anspruch 3, wobei die zusammengesetzte Biomarker-Punktzahl (composite biomarker score) Kontrolle ein vorbestimmter Cut-off-Wert ist.

5. Verfahren nach Anspruch 3 oder 4, wobei die Punktzahl (score) für jedes Protein ein Binärwert ist, der auf dem Basislinien-Expressionslevel für jedes der Proteine basiert.

6. Verfahren nach Anspruch 5, wobei der Binärwert 0 oder 1 ist.

7. Verfahren nach einem der Ansprüche 3 bis 6, wobei das bessere therapeutische Ergebnis ein längeres Gesamtüberleben oder ein längeres progressionsfreies Überleben ist.

8. Verfahren nach einem der Ansprüche 3 bis 7, wobei das Nierenzellkarzinom ein fortgeschrittenes Nierenzellkarzinom oder ein metastasiertes Nierenzellkarzinom ist.

9. Verfahren nach einem der Ansprüche 3 bis 8, wobei die mindestens fünf Proteine aus HGF, MIG, IL-18BP, IL-18 und ANG-2 bestehen.

10. Verfahren nach einem der Ansprüche 3 bis 8, wobei die mindestens fünf Proteine aus TIMP-1, M-CSF, IL-18BP, ANG-2 und VEGF-A bestehen.

11. Verfahren zur Vorhersage der Reaktion eines menschlichen Subjekts, das ein Nierenzellkarzinom hat, von dem vermutet wird ein Nierenzellkarzinom zu haben, oder das gefährdet ist ein Nierenzellkarzinom zu entwickeln, auf eine Kombinationstherapie, die Lenvatinib oder ein pharmazeutisch akzeptables Salz davon umfasst, vorzugsweise Lenvatinibmesylat und Everolimus, das Verfahren umfassend die Schritte:
Messen oder gemessen haben der Basislinien-Expressionslevel von mindestens zwei Proteinen, die IL-18BP und ANG-2 umfassen, in einer biologischen Probe erhalten von dem menschlichen Subjekt,
wobei vorzugsweise das Basislinien-Expressionslevel jedes der Proteine durch Messen der Menge jedes der Proteine in der biologischen Probe bestimmt wird oder das Basislinien-Expressionslevel jedes der Proteine durch Messen der Menge von mRNAs, die für jedes der Proteine kodieren, in der biologischen Probe bestimmt wird;
Feststellen oder festgestellt haben einer Punktzahl (score) für jedes Protein aufgrund seines Basislinien-Expressionslevels;
Aufsummieren oder aufsummiert haben der Punktzahlen (scores), um eine zusammengesetzte Biomarker-Punktzahl (composite biomarker score) zu erhalten; und
wobei
(a) die Punktzahl (score) für jedes Protein größer ist, wenn das Basislinien-Expressionslevel in einen Wertebereich fällt, der mit einer Population menschlicher Subjekte mit besserem therapeutischen Ergebnis als Reaktion auf die Kombinationstherapie korreliert ist, und die zusammengesetzte Biomarker-Punktzahl (composite biomarker score) gleich oder höher als eine zusammengesetzte Biomarker-Punktzahl (composite biomarker score) Kontrolle prädiktiv dafür ist, dass das menschliche Subjekt auf die Kombinationstherapie anspricht, die Lenvatinib oder ein pharmazeutisch akzeptables Salz davon umfasst, vorzugsweise Lenvatinibmesylat und Everolimus; oder
(b) die Punktzahl (score) für jedes Protein kleiner ist, wenn das Basislinien-Expressionslevel in einen Wertebereich fällt, der mit einer Population menschlicher Subjekte mit besserem therapeutischen Ergebnis als Reaktion auf die Kombinationstherapie korreliert ist, und die zusammengesetzte Biomarker-Punktzahl (composite biomarker score) gleich oder niedriger als eine zusammengesetzte Biomarker-Punktzahl (composite biomarker score) Kontrolle prädiktiv dafür ist, dass das menschliche Subjekt auf die Kombinationstherapie anspricht, die Lenvatinib oder ein pharmazeutisch akzeptables Salz davon umfasst, vorzugsweise Lenvatinibmesylat und Everolimus,
wobei die zusammengesetzte Biomarker-Punktzahl (composite biomarker score) Kontrolle eine zusammengesetzte Biomarker-Punktzahl (composite biomarker score) aus einer biologischen Probe erhalten von einem Subjekt ist, das nicht auf die Kombinationstherapie anspricht, die Lenvatinib oder ein pharmazeutisch akzeptables Salz davon umfasst, vorzugsweise Lenvatinibmesylat und Everolimus, und
wobei die biologische Probe eine Blutprobe ist, vorzugsweise eine Serumprobe, oder eine Plasmaprobe.

12. Verfahren nach Anspruch 11, wobei die zusammengesetzte Biomarker-Punktzahl (composite biomarker score) Kontrolle ein vorbestimmter Cut-off-Wert ist.

13. Verfahren nach Anspruch 11 oder 12, wobei die Punktzahl (score) für jedes Protein ein Binärwert ist, der auf dem Basislinien-Expressionslevel für jedes der Proteine basiert.

14. Verfahren nach Anspruch 13, wobei der Binärwert 0 oder 1 ist.

15. Verfahren nach Anspruch 13 oder 14, wobei das bessere therapeutische Ergebnis ein längeres Gesamtüberleben oder ein längeres progressionsfreies Überleben ist.

16. Verfahren nach einem der Ansprüche 11 bis 15, wobei das Nierenzellkarzinom ein fortgeschrittenes Nierenzellkarzinom oder ein metastasiertes Nierenzellkarzinom ist.

17. Verfahren nach einem der Ansprüche 11 bis 16, wobei die mindestens zwei Proteine aus IL-18BP und ANG-2 bestehen.

## Revendications

1. Procédé de prédiction de la réponse d'un sujet humain présentant, ou étant suspecté de présenter, ou étant à risque de développer un hypernéphrome à une polythérapie comprenant du lenvatinib ou un sel pharmaceutiquement acceptable de celui-ci, de préférence du mésylate de lenvatinib ou de l'évérolimus, le procédé comprenant le fait de mesure ou d'avoir fait mesurer un niveau d'expression de base d'au moins une protéine sélectionnée dans le groupe consistant en IL-18BP, ICAM-1, FGF-21 et M-CSF dans un échantillon biologique obtenu à partir du sujet humain,
dans lequel de préférence le niveau d'expression de base d'au moins une protéine est déterminé en mesurant la quantité d'au moins une protéine dans l'échantillon biologique ou le niveau d'expression de base d'au moins une protéine est déterminé en mesurant la quantité d'ARNm codant pour la au moins une protéine dans l'échantillon biologique,
dans lequel un niveau d'expression de base plus faible de la protéine, par rapport à un niveau d'expression de contrôle, de la au moins une protéine, permet de prédire que le sujet humain est sensible à la polythérapie comprenant le lenvatinib ou un sel pharmaceutiquement acceptable de celui-ci, de préférence le mésylate de lenvatinib et l'évérolimus,
dans lequel le niveau d'expression de contrôle est un niveau d'expression dans un échantillon biologique obtenu à partir d'un sujet qui n'est pas sensible à la polythérapie comprenant le lenvatinib ou un sel pharmaceutiquement acceptable de celui-ci, de préférence le mésylate de lenvatinib et l'évérolimus, et
dans lequel l'échantillon biologique est un échantillon de sang, de préférence un échantillon de sérum, ou un échantillon de plasma.

2. Procédé selon la revendication 1, dans lequel l'hypernéphrome est un hypernéphrome avancé ou un hypernéphrome métastatique.

3. Procédé de prédiction de la réponse d'un sujet humain présentant, ou étant suspecté de présenter, ou étant à risque de développer un hypernéphrome à une polythérapie comprenant du lenvatinib ou un sel pharmaceutiquement acceptable de celui-ci, de préférence le mésylate de lenvatinib et l'évérolimus, le procédé comprenant les étapes consistant à :
mesurer ou faire mesurer les niveaux d'expression de base d'au moins cinq protéines dans un échantillon biologique obtenu à partir du sujet humain,
dans lequel de préférence le niveau d'expression de base des au moins cinq protéines est déterminé en mesurant la quantité des au moins cinq protéines dans l'échantillon biologique ou le niveau d'expression de base des au moins cinq protéines est déterminé en mesurant la quantité d'ARNm codant pour les au moins cinq protéines dans l'échantillon biologique ;
déterminer un score pour chaque protéine en fonction de son niveau d'expression de base ;
additionner les scores pour obtenir un score composite des biomarqueurs ; et
déterminer que le sujet humain est sensible à la polythérapie sur la base du score composite des biomarqueurs ;
dans lequel les au moins cinq protéines comprennent
(i) HGF, MIG, IL-18BP, IL-18 et ANG-2; ou
(ii) TIMP-1, M-CSF, IL-18BP, ANG-2 et VEGF; et
dans lequel
(a) le score pour chaque protéine est plus grand si le niveau d'expression de base tombe dans une plage de valeurs qui est corrélée avec une population de sujets humains présentant un meilleur résultat thérapeutique en réponse à la polythérapie, et le score composite des biomarqueurs égal ou supérieur à un score composite des biomarqueurs de contrôle permet de prédire que le sujet humain répond à la polythérapie comprenant le lenvatinib ou un sel pharmaceutiquement acceptable de celui-ci, de préférence le mésylate de lenvatinib et l'évérolimus ; ou
(b) le score pour chaque protéine est plus petit si le niveau d'expression de base tombe dans une plage de valeurs qui est corrélée avec une population de sujets humains présentant un meilleur résultat thérapeutique en réponse à la polythérapie, et le score composite des biomarqueurs égal ou inférieur à un score composite des biomarqueurs de contrôle permet de prédire que le sujet humain répond à la polythérapie comprenant le lenvatinib ou un sel pharmaceutiquement acceptable de celui-ci, de préférence le mésylate de lenvatinib et l'évérolimus,
dans lequel le score composite des biomarqueurs est un score composite des biomarqueurs d'un échantillon biologique obtenu à partir d'un sujet qui n'est pas sensible à la polythérapie comprenant le lenvatinib ou un sel pharmaceutiquement acceptable de celui-ci, de préférence le mésylate de lenvatinib et l'évérolimus, et
dans lequel l'échantillon biologique est un échantillon de sang, de préférence un échantillon de sérum, ou un échantillon de plasma.

4. Procédé selon la revendication 3, dans lequel le score composite des biomarqueurs est une valeur de coupure prédéterminée.

5. Procédé selon la revendication 3 ou 4, dans lequel le score pour chaque protéine est une valeur binaire basée sur le niveau d'expression de base pour chacune des protéines.

6. Procédé selon la revendication 5, dans lequel la valeur binaire est 0 ou 1.

7. Procédé selon l'une quelconque des revendications 3 à 6, dans lequel le meilleur résultat thérapeutique est une survie globale plus longue ou une survie sans progression plus longue.

8. Procédé selon l'une quelconque des revendications 3 à 7, dans lequel l'hypernéphrome est un hypernéphrome avancé ou un hypernéphrome métastatique.

9. Procédé selon l'une quelconque des revendications 3 à 8, dans lequel les au moins cinq protéines consistent en HGF, MIG, IL-18BP, IL-18 et ANG-2.

10. Procédé selon l'une quelconque des revendications 3 à 8, dans lequel les au moins cinq protéines consistent en TIMP-1, M-CSF, IL-18BP, ANG-2 et VEGF-A.

11. Procédé de prédiction de la réponse d'un sujet humain présentant, ou étant suspecté de présenter, ou étant à risque de développer un hypernéphrome à une polythérapie comprenant du lenvatinib ou un sel pharmaceutiquement acceptable de celui-ci, de préférence le mésylate de lenvatinib et l'évérolimus, le procédé comprenant les étapes consistant à :
mesurer ou faire mesurer les niveaux d'expression de base d'au moins deux protéines comprenant IL-18BP et ANG-2 dans un échantillon biologique obtenu à partie d'un sujet humain,
dans lequel de préférence le niveau d'expression de base de chacune des protéines est déterminé en mesurant la quantité de chacune des protéines dans l'échantillon biologique ou le niveau d'expression de base de chacune des protéines est déterminé en mesurant la quantité d'ARNm codant de chacune des protéines dans l'échantillon biologique ;
déterminer ou avoir déterminé un score pour chaque protéine en fonction de son niveau d'expression de base ;
additionner ou avoir additionné les scores pour obtenir un score composite des biomarqueurs ; et
dans lequel
(a) le score pour chaque protéine est plus grand si le niveau d'expression de base tombe dans une plage de valeurs qui est corrélée avec une population de sujets humains présentant un meilleur résultat thérapeutique en réponse à la polythérapie, et le score composite des biomarqueurs égal ou supérieur à un score composite des biomarqueurs de contrôle permet de prédire que le sujet humain répond à la polythérapie comprenant le lenvatinib ou un sel pharmaceutiquement acceptable de celui-ci, de préférence le mésylate de lenvatinib et l'évérolimus ; ou
(b) le score pour chaque protéine est plus petit si le niveau d'expression de base tombe dans une plage de valeurs qui est corrélée avec une population de sujets humains présentant un meilleur résultat thérapeutique en réponse à la polythérapie, et le score composite des biomarqueurs égal ou inférieur à un score composite des biomarqueurs de contrôle permet de prédire que le sujet humain répond à la polythérapie comprenant le lenvatinib ou un sel pharmaceutiquement acceptable de celui-ci, de préférence le mésylate de lenvatinib et l'évérolimus,
dans lequel le score composite des biomarqueurs est un score composite des biomarqueurs d'un échantillon biologique obtenu à partir d'un sujet qui n'est pas sensible à la polythérapie comprenant le lenvatinib ou un sel pharmaceutiquement acceptable de celui-ci, de préférence le mésylate de lenvatinib et l'évérolimus, et
dans lequel l'échantillon biologique est un échantillon de sang, de préférence un échantillon de sérum, ou un échantillon de plasma.

12. Procédé selon la revendication 11, dans lequel le score composite des biomarqueurs est une valeur de coupure prédéterminée.

13. Procédé selon la revendication 11 ou 12, dans lequel le score pour chaque protéine est une valeur binaire basée sur le niveau d'expression de base pour chacune des protéines.

14. Procédé selon la revendication 13, dans lequel la valeur binaire est 0 ou 1.

15. Procédé selon la revendication 13 ou 14, dans lequel le meilleur résultat thérapeutique est une survie globale plus longue ou une survie sans progression plus longue.

16. Procédé selon l'une quelconque des revendications 11 à 15, dans lequel l'hypernéphrome est un hypernéphrome avancé ou un hypernéphrome métastatique.

17. Procédé selon l'une quelconque des revendications 11 à 16, dans lequel au moins deux protéines consistent en IL-18BP et ANG-2.
